(19) Europäisches Patentamt
European Patent Office
Office européen des brevets

(11) Publication number: **0 549 079 A1**

(12) **EUROPEAN PATENT APPLICATION**

(21) Application number: **92204080.3**

(22) Date of filing: **23.12.92**

(51) Int. Cl.⁵: **C07D 239/60**, C07D 239/553, C07D 239/34, C07D 251/30, C07D 251/20, C07D 417/12, C07D 401/12, A01N 43/54, A01N 43/66

(30) Priority: **24.12.91 EP 91122208**

(43) Date of publication of application: **30.06.93 Bulletin 93/26**

(84) Designated Contracting States: **AT BE CH DE DK ES FR GB GR IT LI LU NL SE**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Newton, Trevor William**
**Friedensstrasse 20**
**W-6501 Schwabenheim(DE)**

(54) **Sulphonamide herbicides.**

(57) Compounds of the formula

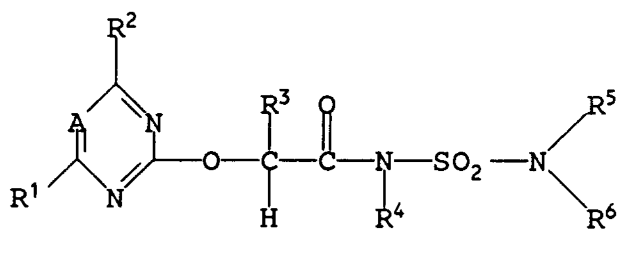

(I)

in which

A is nitrogen or $CR^7$;

$R^1$, $R^2$ and $R^7$ each independently are hydrogen, halogen, formyl, cyano, carboxy or azido, or optionally substituted alkyl, alkenyl, alkynyl, cycloalkyl, alkoxy, alkenyloxy, alkynyloxy, aryloxy, alkylthio, alkenylthio, alkynylthio, arylthio, alkylcarbonyl, alkoxycarbonyl, amino. aminoxy or dialkyliminoxy;

$R^3$ is hydrogen, or optionally substituted alkyl, alkenyl, alkynyl, cycloalkyl, heterocyclic, aralkyl or aryl;

$R^4$ is hydrogen or optionally substituted alkyl, alkenyl, aralkyl, aryl, heterocyclic or acyl; and

$R^5$ and $R^6$ each independently are hydrogen or optionally substituted alkyl, alkoxy, alkenyl, alkynyl, aryl, aralkyl, amino, cycloalkyl or heterocyclic, or a sulphonyl-containing group, or

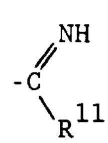

EP 0 549 079 A1

where $R^{11}$ is alkylthio, or together form

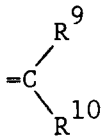

where $R^9$ and $R^{10}$ each independently are hydrogen, alkyl, alkoxy, aryl, aralkyl or dialkylamino, or $R^9$ and $R^{10}$ together form optionally substituted heterocyclic or optionally interrupted alkylene chain; and salts thereof, have herbicidal properties.

The present invention relates to certain new sulphonamide derivatives, their preparation, herbicidal compositions containing them, and their use in combating undesired plant growth.

Sulphonamide compounds are well known for their biological activity. Certain classes of sulphonamide derivatives are useful as herbicides whilst other classes are useful as anti-bacterial agents.

In EP-A-0411706 it is disclosed that sulphonamide compounds of the general formula

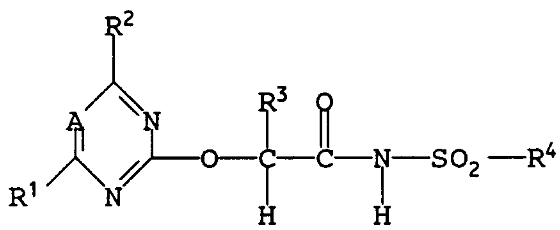

in which A is a nitrogen atom or a group $CR^5$, each of $R^1$, $R^2$, $R^3$ and $R^5$ is one of a range of moieties, and, in particular, $R^4$ is an optionally substituted alkyl, aralkyl, aryl or heterocyclic group, or salts thereof, exhibit herbicidal activity.

A class of sulphonamide derivatives has now been found which differ structurally from those disclosed in EP-A-0411706 in that the terminal group $R^4$ has been replaced by a substituted amino group and which have useful herbicidal properties.

The present invention thus provides a compound of the general formula

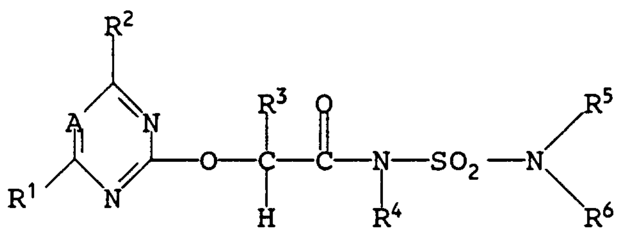

(I)

in which

| | |
|---|---|
| A | represents a nitrogen atom or a group $CR^7$; |
| $R^1$, $R^2$ and $R^7$ | each independently represents a hydrogen or halogen atom, a formyl, cyano, carboxy or azido group, or an optionally substituted alkyl, alkenyl, alkynyl, cycloalkyl, alkoxy, alkenyloxy, alkynyloxy, aryloxy, alkylthio, alkenylthio, alkynylthio, arylthio, alkylcarbonyl, alkoxycarbonyl, amino, aminoxy or dialkyliminoxy group; |
| $R^3$ | represents a hydrogen atom, or an optionally substituted alkyl, alkenyl, alkynyl, cycloalkyl, heterocyclic, aralkyl or aryl group; |
| $R^4$ | represents a hydrogen atom, or an optionally substituted alkyl, alkenyl, aralkyl, aryl or heterocyclic group, or an optionally substituted acyl group of the formula $COR^8$ in which $R^8$ represents an alkyl, aryl or aralkyl group; and |
| $R^5$ and $R^6$ | each independently represents a hydrogen atom or an optionally substituted alkyl, alkoxy, alkenyl, alkynyl, aryl, aralkyl, amino, cycloalkyl or heterocyclic group, or a group of the formula $SO_2R^8$, in which $R^8$ is as defined hereinabove, or a group |

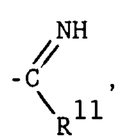

in which $R^{11}$ is an alkylthio group, or together form a group

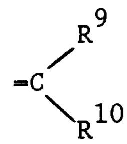

in which $R^9$ and $R^{10}$ each independently represents a hydrogen atom or an alkyl, alkoxy, aryl, aralkyl or dialkylamino group, or $R^9$ and $R^{10}$ together form an optionally substituted heterocyclic group, or $R^5$ and $R^6$ together form an alkylene chain which is optionally interrupted by an oxygen or sulphur atom, or by a group -NR-in which R represents a hydrogen atom or an alkyl group;

or a salt thereof.

An alkyl, alkenyl or alkynyl radical or moiety may be a straight or branched chain group. Generally an alkyl radical or moiety has from 1 to 12 carbon atoms, preferably from 1 to 6, especially from 1 to 4, carbon atoms. Alkenyl and alkynyl radicals or moieties suitably have from 2 to 12 carbon atoms, preferably from 2 to 6, especially from 2 to 4, carbon atoms. Cycloalkyl groups suitably have from 3 to 8 carbon atom ring members.

An aryl radical, or an aryl moiety in an aralkyl, aryloxy, arylthio or acyl radical, may be a single or fused carbocyclic ring system having from 6 to 10 ring members. Suitably an aryl radical or moiety comprises a single ring system and preferably is a phenyl ring.

A heterocyclic radical is suitably a single or fused, saturated or unsaturated ring system having from 5 to 10, preferably 5 or 6, ring members of which from 1 to 3 ring members may be hetero atoms selected from oxygen, nitrogen and sulphur atoms.

Radicals represented by the symbols $R^1$, $R^2$, $R^3$, $R^4$, $R^5$, $R^6$, $R^7$, $R^8$, $R^9$ and $R^{10}$ may be unsubstituted or substituted. Where substituents are present, the substituent groups may be any of those customarily employed in the modification and/or development of pesticidal compounds and are especially substituents that maintain or enhance the herbicidal activity associated with the compounds of the present invention, or influence persistence of action, soil or plant penetration, or any other desirable property for herbicidal compounds. There may be one or more of the same or different substituents present in each radical.

Optional substituents for alkyl, alkenyl, alkynyl, alkoxy, alkenyloxy, alkynyloxy, alkylthio, alkenylthio, alkynylthio, alkylcarbonyl and alkoxycarbonyl groups or alkyl moieties in aralkyl groups or alkyl moieties in acyl groups may be independently selected from one or more of halogen atoms and alkoxy, alkenyloxy, aryloxy, hydroxy, alkylthio, arylthio, aryl, alkylsulphonyl, alkylsulphinyl, alkylenedioxy, alkylenedithio, haloalkyl and alkoxycarbonyl groups, heterocyclic groups, and dialkyliminoxy, optionally substituted amino, trialkylsilyl, alkylcarbonyl, arylcarbonyl, alkoxycarbonyl, carboxy, cyano, thiocyanato and optionally substituted aminocarbonyl groups.

Optional substituents for aryl, cycloalkyl aryloxy or arylthio groups, heterocyclic rings or aryl moieties in aralkyl groups may be independently selected from one or more of halogen atoms and nitro, cyano, alkyl, haloalkyl, alkoxy, haloalkoxy, alkylthio, alkylsulphonyl, mono- or di-alkylsulphonamido, aryloxy, carboxy, alkoxycarbonyl and aralkoxycarbonyl groups.

Optional substituents for an amino group or for an amino moiety in an aminoxy or aminocarbonyl group, may suitably be independently selected from alkyl, alkenyl, aryl, alkoxy, amino, mono- or di-alkylamino, arylamino, alkoxyalkyl, haloalkyl, hydroxy, hydroxyalkyl, cyano, carboxyalkyl or alkylcarbonylamino, or the amino group may form part of a heterocyclic ring.

Suitable salts of the invention are agrochemically acceptable salts of compounds of general formula I. It is possible for salts to be formed with inorganic or organic cations by conventional methods. Such salts suitably include salts with inorganic cations derived from alkali metals and alkaline earth metals such as, for example, sodium, potassium, calcium and magnesium, and from transition metals, for example copper, and salts with organic cations such as alkylammonium and alkylsulphonium cations.

A haloalkyl or haloalkoxy radical suitably has from 1 to 3 halogen atoms; a preferred haloalkyl radical is a trifluoroethyl group. A halogen atom as a substituent is suitably a fluorine, chlorine or bromine atom.

A is preferably a nitrogen atom or a group CH.

Suitable examples of the radical $R^1$ include $C_{1-4}$ alkyl groups, $C_{1-4}$ alkoxy groups and $C_{1-4}$ haloalkyl groups, preferably methyl, methoxy and trifluoromethyl groups. Suitable examples of the radical $R^2$ include $C_{1-4}$ alkyl groups, $C_{1-4}$ alkoxy groups and halogen atoms, preferably methyl and methoxy groups and chlorine atoms.

Suitable examples of the radical $R^3$ include $C_{1-6}$ alkyl and phenyl groups. Preferably the radical $R^3$ is selected from methyl, ethyl, n-propyl, i-propyl, n-butyl, i-butyl, s-butyl, t-butyl and phenyl groups.

Preferably, $R^4$ represents a hydrogen atom.

Suitably, $R^5$ and $R^6$ each independently represents a hydrogen atom, a $C_{1-6}$ alkyl, $C_{1-6}$ haloalkyl, $C_{1-6}$ alkoxy, $C_{2-6}$ alkenyl,$C_{2-6}$ alkynyl, mono- or di-($C_{1-4}$ alkoxy)$C_{1-4}$ alkyl, ($C_{1-4}$ alkoxy)carbonyl($C_{1-4}$ alkyl), $C_{3-8}$ cycloalkyl or benzyl group, or an optionally substituted phenyl, pyridyl, pyrimidinyl or ($C_{3-8}$ cycloalkyl)$C_{1-4}$ alkyl group,
or a group

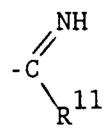

in which $R^{11}$ is a $C_{1-4}$ alkylthio group, or together form a group

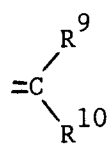

in which $R^9$ and $R^{10}$ each independently represents a $C_{1-4}$ alkyl, $C_{1-4}$ alkoxy or di($C_{1-4}$ alkyl)amino group, or $R^9$ and $R^{10}$ together form a five-membered ring in which two or three ring members are hetero atoms selected from nitrogen and sulphur atoms, the ring being substituted by a benzyl or one or two $C_{1-4}$ alkyl groups, or $R^5$ and $R^6$ together form an alkylene chain which is optionally interrupted by an oxygen atom or by a group -NR- in which R represents a $C_{1-4}$ alkyl group.

Preferably $R^5$ represents a hydrogen atom or a methyl or ethyl group, $R^6$ represents a hydrogen atom or a methyl, ethyl, n-propyl, i-propyl, n-butyl, methoxy, chloroethyl, methoxycarbonylmethyl, mono- or di-methoxyethyl, allyl, propynyl, cyclopropyl, cyclobutyl, pyridyl, dimethylpyrimidinyl, (dichlorocyclopropyl)-methyl, phenyl, chlorophenyl or benzyl group or a group

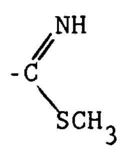

,

or $R^5$ and $R^6$ together represent one of the groups

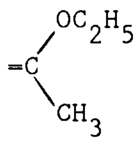 , 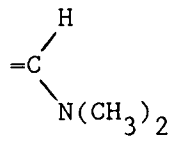 , 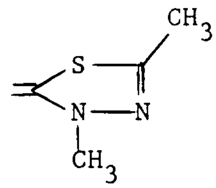 ,

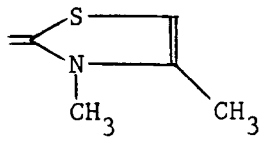 ,

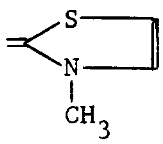 and 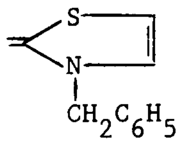 ,

or $R^5$ and $R^6$ together represent a group $-(CH_2)_2O(CH_2)_2-$, $-(CH_2)_2N(CH_3)(CH_2)_2-$ or $-(CH_2)_4-$.

It will be appreciated that the compounds of the present invention in which $R^3$ is other than a hydrogen atom have an asymmetric carbon atom and will therefore exist in different stereoisomeric forms. The present invention accordingly includes all individual isomeric forms of the compounds of general formula I and mixtures thereof in whatever proportion. Thus, the R- and S-enantiomers of the compound of general formula IA

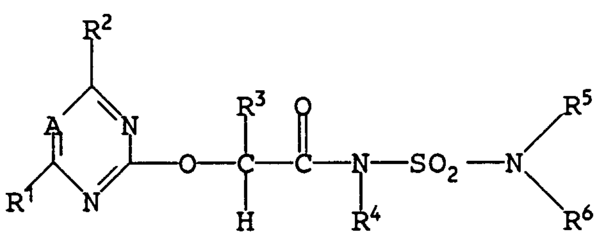 (IA)

in which A, $R^1$, $R^2$, $R^4$, $R^5$ and $R^6$ are as hereinbefore defined and $R^3$ is other than a hydrogen atom, and mixtures thereof, are included within the present invention.

In accordance with the present invention there is also provided a process for the preparation of a compound of the general formula I, which process comprises

(a) reacting a compound of the general formula

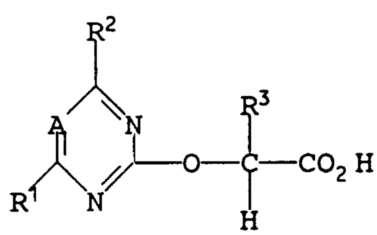 (II)

in which A, $R^1$, $R^2$ and $R^3$ are as defined above, or a corresponding ester, acid chloride or acid anhydride, with a compound of the general formula

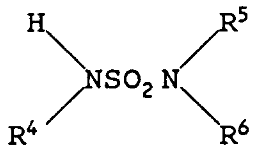

(III)

in which $R^4$, $R^5$ and $R^6$ are as previously defined, or a salt thereof, if appropriate in the presence of a carboxyl-activating agent,
or
(b) reacting a compound of the general formula

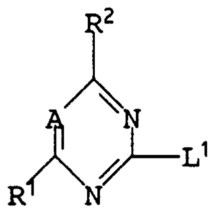

(IV)

in which A, $R^1$ and $R^2$ are as previously defined and $L^1$ represents a leaving group, with, when $R^4$ represents a hydrogen atom, a di-salt, and, when $R^4$ represents a moiety other than a hydrogen atom, a mono-salt of a compound of the general formula

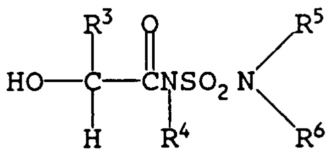

(V)

in which $R^3$, $R^4$, $R^5$ and $R^6$ are as previously defined,
and, if required or desired, converting a resulting compound into any other compound of the invention.

A leaving group is any group that will, under the reaction conditions, cleave from the starting material thus promoting reaction at a specified site.

The leaving group $L^1$ is conveniently a halogen atom, for example a bromine, chlorine or iodine atom, or, especially for the pyrimidine starting materials, an alkanesulphonyl group, for example methanesulphonyl.

A salt of compound V is suitably an alkali metal salt, preferably a sodium salt.

Process (a) is suitably carried out at ambient or elevated temperature, i.e. at a temperature above 20 °C. A preferred temperature range in which to carry out the reaction is from 20 °C to 80 °C; an especially suitable reaction temperature is in the range of from 20 °C to 50 °C. The molar ratio of reactant II to reactant III may, for example, be in the range of from 1.0 to 5.0 preferably from 1.0 to 2.5.

The reaction (a) is suitably carried out in an inert organic solvent such as a hydrocarbon solvent, e.g. benzene or toluene, a chlorinated hydrocarbon, e.g. dichloromethane or chloroform, an alcohol, e.g. methanol or ethanol, an ether, e.g. diethyl ether, tetrahydrofuran, 1,4-dioxane, a ketone, e.g. acetone or methyl ethyl ketone, an ester, e.g. ethyl acetate, an aprotic polar solvent, e.g. dimethylformamide, dimethylacetamide or dimethylsulphoxide or a nitrile, e.g. acetonitrile.

Preferably, the reaction (a) is carried out in the presence of a tertiary amine, for example 1,8-diazabicyclo[5.4.0]undec-7-ene. Other suitable tertiary amines include triethylamine and pyridine.

When the reactant II is in the form of a free carboxylic acid, the carboxy group needs to be activated for the reaction to proceed. Suitable carboxyl-activating agents include 2-chloro-N-methyl pyridinium iodide, dicylohexylcarbodiimide and carbonyldiimidazole. Suitably the acid reactant II is activated by the carboxyl-activating agent in the presence of an inert organic solvent at ambient or elevated temperature, for example at a temperature in the range of from 20 °C to the reflux temperature of the mixture, prior to the addition of reactant III and, if desired, the tertiary amine.

Process (b) is suitably carried out at a temperature in the range of from ambient to the reflux temperature of the reaction medium, preferably in the range of from 100 to 150 °C, for example at 120 °C. The molar ratio of the reactants IV:V is suitably in the range of from 1.0 to 2.5.

In reaction (b) the salt may suitably be prepared from a compound V by the action of an alkali metal, such as metallic sodium or potassium, or, conveniently, a strong base, for example, an alkali metal hydride, such as sodium or potassium hydride, an alkaline earth metal hydride, such as calcium hydride, an alkali metal alkoxide, such as potassium t-butoxide, or an alkali metal hydroxide, such as sodium or potassium hydroxide. Suitably conversion of a hydroxy compound V to the salt occurs in situ.

Suitably, the reaction (b) is carried out in the presence of a solvent; typical solvents are, for example, the same as noted above for process (a).

The compound of general formula I obtained by either of the methods (a) or (b) may be converted to a further compound of general formula I by methods known to a man skilled in the art, provided that suitable care is taken to ensure that the sulphonamide group is not affected. Thus for example, a compound of general formula I where $R^1$ and/or $R^2$ represents a halogen atom, suitably chlorine, may be transformed into other derivatives by nucleophilic displacement, for example by reaction with two equivalents of an amine, such as dimethylamine, to give the corresponding compound of general formula I in which $R^1$ and/or $R^2$ represents a substituted amino group. Likewise a compound of general formula I in which $R^1$ and/or $R^2$ represents a halogen atom, may be reacted with two equivalents of an alkylthio organo-metallic compound, for example sodium methanethiolate, to yield the corresponding compound of general formula I in which $R^1$ and/or $R^2$ represents an alkylthio group such as methylthio, or may be hydrogenated to yield the corresponding compound in which $R^1$ and/or $R^2$ is a hydrogen atom. Furthermore, a compound of general formula I in which $R^4$ represents a hydrogen atom can be converted into the corresponding compound of formula I in which $R^4$ represents a moiety other than a hydrogen atom.

Acid and salt conversion reactions may be carried out using conventional techniques as appropriate.

Individual enantiomers may be obtained using stereospecific reactants or by conventional resolution techniques.

The prepared compounds of the invention may, if desired, be isolated and purified using conventional techniques.

Suitable starting carboxylic acids of general formula II, and esters thereof, and also their preparation, are described and claimed in EP-A-0400741. Thus the starting carboxylic acids of general formula II, and esters thereof, may be prepared either by reacting a compound of the general formula

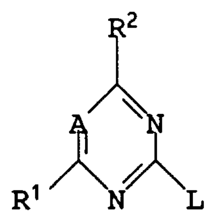

(VI)

in which $R^1$, $R^2$ and A are as defined above and L represents a leaving group, for example a halogen atom or alkanesulphonyl group, with a compound of the general formula

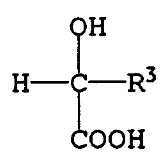

(VII)

in which $R^3$ is as defined above, or an ester thereof,

or

for compounds in which A represents $CR^7$, reacting a compound of the general formula

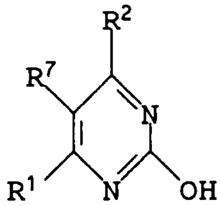

(VIII)

in which $R^1$, $R^2$ and $R^7$ are as defined above, preferably with an ester of a compound of the general formula

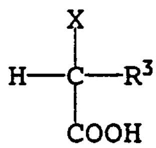

(IX)

in which $R^3$ is as defined above and X represents a leaving group, for example a halogen atom or a sulphonyloxy group, and, if required or desired, converting the resulting ester into another ester or into a corresponding acid, or converting an acid into another acid or into an ester. The acid chloride and acid anhydride derivatives are preparable from the compounds of formula II by standard techniques.

The reactants of general formula III are either known or can be prepared using techniques described in the literature. For example, said compounds may be prepared by methods such as those described in (A) G. Lohaus, Chem. Ber., 105, 2791 (1972) or in (B) G. Weiss and G. Schulze, Liebigs Ann. Chem., 729, 40 (1969)

The starting triazine compounds of general formulae IV and VI (i.e. in which A is a nitrogen atom) are either known or can be prepared using techniques described in the literature. For example such compounds may be prepared from 2,4,6-trichloro- triazine by methods such as those described by Dudley et al, J. Am. Chem. Soc., 73, 2986, (1951), Koopman et al, Rec. Trav. Chim., 79, 83, (1960), Hirt et al, Helv. Chim. Acta, 33, 1365, (1950), Kobe et al, Monatshefte fur Chemie, 101, 724, (1970) and Ross et al, US Patent Specification No. 3 316 263.

The starting pyrimidines of general formulae IV, VI and VIII (i.e. in which A is a group CH) may be prepared by conventional techniques, for example those described in Heterocyclic compounds, 16 "The Pyrimidines", edited by D.J. Brown, Interscience, 1962.

The compounds of general formula V may be prepared from the corresponding benzyloxy derivatives

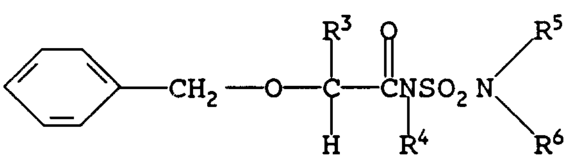

(X)

by hydrogenation, suitably using gaseous hydrogen in conjunction with a palladium- or platinum- carbon catalyst. The benzyloxy derivatives may be prepared in analogous fashion to reaction (a) above by the reaction of an appropriate 2-benzyloxycarboxylic acid

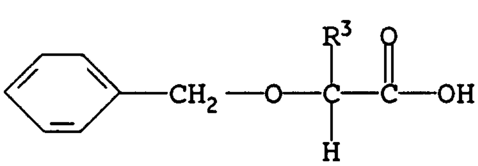

(XI)

or reactive derivative thereof, with a compound of general formula III described above, or a salt thereof.

The compounds of general formula VII are either known compounds or may be prepared by conventional procedures. Compounds in which $R^3$ represents an aryl group may for example be prepared by treating the corresponding aldehyde, $R^3CHO$, with a suitable cyanide compound, for example potassium cyanide or trimethylsilylcyanide with, respectively, zinc iodide or sodium bisulphite, followed by conversion of the cyano substituent to the acid group, see, for example, Schnur and Morville, J.Med. Chem. 29, 770 (1986) and U.S. Patent Specification No. 4 537 623. Compounds in which $R^3$ represents an alkyl group may, for example, be prepared by the method of Kolasa and Miller, J. Org. Chem. 52, 4978, (1987), starting from a suitable amino acid with a 2 stage conversion.

The compounds of general formula IX may be prepared by conventional techniques, for example by halogenating a corresponding compound, for example by the procedure of Epstein et al. J.Med. Chem., 24, 481, (1981).

Compounds of the general formula I have been found to have useful herbicidal activity. Accordingly the present invention further provides a herbicidal composition comprising a compound of formula I as defined above in association with at least one carrier.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing $\frac{1}{2}$-10% w of active ingredient. Granules are usually prepared to have a size between 10 and 100 BS mesh (1.676 - 0.152 mm), and may be manufactured by agglomeration or

impregnation techniques. Generally, granules will contain ½-75% w active ingredient and 0-10% w of additives such as stabilisers, surfactants, slow release modifiers and binding agents. The so-called "dry-flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other active ingredients, for example compounds possessing insecticidal or fungicidal properties or other herbicides.

The present invention still further provides the use as a herbicide of a compound of the general formula I as defined above or a composition as defined above and a method of combating undesired plant growth at a locus with such a compound or composition according to the present invention. The locus may, for example, be the soil or plants in a crop area. The dosage of active ingredient used, may, for example, be in the range of from 0.01 to 10 kg/ha, suitably 0.05 to 4 kg/ha.

Examples 1-166 below illustrate the process of the present invention; Examples 1 and 2 illustrate the preparation of intermediates of general formula III, by Methods (A) and (B) respectively, while Examples 3 to 166 illustrate the preparation of compounds of general formula I.

EXAMPLE 1

Preparation of 1-n-butylsulphamide

(i) A solution of chlorosulphonylisocyanate (60.9 g, 0.43 mol) in toluene (20 ml) was added dropwise to a stirred solution of 2,4,5-trichlorophenol (84.9 g, 0.43 mol) in toluene (80 ml), maintaining the temperature of the reaction mixture at or below 35 °C. After the addition was complete, the mixture was refluxed for 3 hours, during which, copious hydrogen chloride was evolved. The mixture was then cooled to 40 °C and water was added dropwise until no further carbon dioxide was evolved. The white precipitate which formed was collected by filtration and dried to give the crude O-(2,4,5-trichlorophenyl)- sulphamate as white crystals (93.0 g, 78%), m.p. 153-155 °C, which was used without further purification.

(ii) O-(2,4,5-trichlorophenyl)-sulphamate (13.83 g, 50 mmol) was added in two portions to a stirred solution of n-butylamine (4.94 ml, 50 mmol) and triethylamine (6.9 ml, 50 mmol) in acetonitrile (50 ml). After stirring for 5 min, the solution was evaporated in vacuo. The residue was dissolved in ethyl acetate (50 ml) and the organic phase was washed with 0.05N hydrochloric acid (2 x 25 ml). The organic phase was then dried (sodium sulphate) and evaporated in vacuo. The residue was purified by flash chromatography (ethyl acetate/petroleum ether, 40:60, followed by pure ethyl acetate) to give the title compound as a pale brown waxy solid (2.52 g, 33%).

EXAMPLE 2

Preparation of 1-methylsulphamide

(i) Sulphuryl chloride (81.3 ml, 1.0 mol) and antimony V chloride (0.21 ml, 1.7 mmol) were added sequentially to a stirred suspension of methylamine hydrochloride (67.0 g, 1.0 mol) in acetonitrile (500 ml). The mixture was refluxed for 4 hours, during which time chlorine and hydrogen chloride gases were evolved. Further sulphuryl chloride (81.3 ml, 1.0 mol) was added to the mixture, which was then refluxed again for 4 hours. A final portion of sulphuryl chloride (40.7 ml, 0.5 mol) was then added and the mixture was refluxed overnight. The mixture was then cooled to room temperature and the solvent evaporated in vacuo. The residue was distilled (b.p. 55-57 °C/0.02 mBar) to give methylsulphamoyl chloride as a colourless viscous oil (112 g, 86%).

(ii) Methylsulphamoyl chloride (10.0 g, 77 mmol) was added dropwise slowly to a stirred solution of liquid ammonia (ca. 50 ml, excess) in tetrahydrofuran (250 ml) at -40 °C. A white precipitate was formed. The mixture was allowed to attain room temperature and the excess ammonia and solvent were evaporated in vacuo. The white crystalline residue was recrystallised from ethyl acetate/hexane to give the title compound as white needles (5.53g, 65%), m.p. 62-65 °C.

EXAMPLE 3

Preparation of 1-[2-(4,6-dimethoxypyrimidin-2-yl)oxy-3'3-dimethylbutan-1-oyl]-3-methylsulphamide

A solution 2-(4,6-dimethoxypyrimidin-2-yl-oxy-3,3-dimethylbutan-1-oic acid (1.35 g, 5.0 mmol) in tetrahydrofuran (20 ml) was added dropwise to a stirred solution of carbonyldiimidazole (0.89 g, 5.5 mmol) in tetrahydrofuran (40 ml) at room temperature. The mixture was heated to reflux for 30 min, then allowed to cool again to room temperature. N-methylsulphamide (0.55 g, 5.0 mmol) was added. After stirring the mixture for 15 min, a solution of 1,8-diazabicyclo[5.4.0]undec-7-ene (0.76 g, 5.0 mmol) in tetrahydrofuran (10 ml) was added. After stirring for a further 15 min, the solvent was evaporated off in vacuo. Aqueous sodium chloride solution (40 ml) was added to the residue. The mixture was then acidified with 5N hydrochloric acid until there was no further precipitation. The mixture was extracted with ethyl acetate (4 x 25 ml) and the combined organic phases were dried (sodium sulphate) and evaporated in vacuo. The residue was purified by flash chromatography (dichloromethane/ methanol, 97:3) to give the title compound as white crystals (0.97 g, 54%), m.p. 200-202 °C.

EXAMPLES 4 TO 166

By methods analogous to that of Example 3, further compounds of the general formula I were prepared by reaction of compounds of the general formula II with compounds of general formula III. Details are given in Tables I and II.

Table I

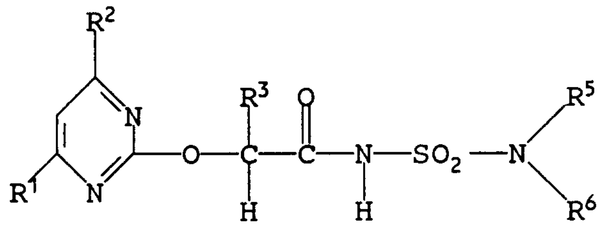

| Ex No | $R^1$ | $R^2$ | $R^3$ | $R^5$ | $R^6$ | mp (°C) | Yield (%) |
|---|---|---|---|---|---|---|---|
| 4 | $OCH_3$ | $OCH_3$ | $tC_4H_9$ | $CH_3$ | $CH_3$ | 164 | 36 |
| 5 | $OCH_3$ | $OCH_3$ | $tC_4H_9$ | H | $C_6H_5$ | 198 | 69 |
| 6 | $OCH_3$ | $OCH_3$ | $tC_4H_9$ | $-(CH_2)_2O(CH_2)_2-$ | | 58 | 39 |
| 7 | $OCH_3$ | $OCH_3$ | $tC_4H_9$ | $-(CH_2)_2N(CH_3)(CH_2)_2-$ | | 225-227 | 19 |
| 8 | $OCH_3$ | $OCH_3$ | $tC_4H_9$ | $-(CH_2)_4-$ | | 145-147 | 55 |
| 9 | $OCH_3$ | $OCH_3$ | $tC_4H_9$ | $CH_3$ | $C_6H_5$ | 134-136 | 40 |
| 10 | $OCH_3$ | $OCH_3$ | $tC_4H_9$ | H | $nC_4H_9$ | 164-167 | 49 |
| 11 | $OCH_3$ | $OCH_3$ | $tC_4H_9$ | H | $CH_2C_6H_5$ | 170-171 | 60 |
| 12 | $OCH_3$ | $OCH_3$ | $iC_4H_9$ | $CH_3$ | $CH_3$ | 115-120 | 76 |
| 13 | $CH_3$ | $CH_3$ | $tC_4H_9$ | $CH_3$ | $CH_3$ | 118-120 | 60 |
| 14 | $CH_3$ | $CH_3$ | $iC_4H_9$ | $CH_3$ | $CH_3$ | 133-136 | 74 |
| 15 | $CH_3$ | $CH_3$ | $C_6H_5$ | $CH_3$ | $CH_3$ | 187 | 36 |
| 16 | $OCH_3$ | $OCH_3$ | $C_6H_5$ | H | $C_6H_5$ | 192-193 | 55 |
| 17 | $OCH_3$ | $OCH_3$ | $C_6H_5$ | $CH_3$ | $C_6H_5$ | 137-138 | 55 |
| 18 | $OCH_3$ | Cl | $iC_4H_9$ | $CH_3$ | $CH_3$ | 150-152 | 41 |
| 19 | $OCH_3$ | Cl | $iC_4H_9$ | H | $CH_3$ | 166-168 | 24 |
| 20 | $CH_3$ | $CH_3$ | $nC_4H_9$ | H | $CH_3$ | 154-156 | 42 |
| 21 | $OCH_3$ | $CH_3$ | $tC_4H_9$ | $CH_3$ | $CH_3$ | 123-124 | 56 |
| 22 | $OCH_3$ | $OCH_3$ | $tC_4H_9$ | H | 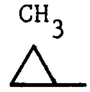 | 188-189 | 73 |
| 23 | $CH_3$ | $CH_3$ | $sC_4H_9$ | $CH_3$ | $CH_3$ | 150 | 60 |

Table I (continued)

| Ex No | $R^1$ | $R^2$ | $R^3$ | $R^5$ | $R^6$ | mp (°C) | Yield (%) |
|---|---|---|---|---|---|---|---|
| 24 | $CH_3$ | $CH_3$ | $sC_4H_9$ | H | $CH_3$ | 161 | 72 |
| 25 | $OCH_3$ | $OCH_3$ | $iC_3H_7$ | $CH_3$ | $CH_3$ | 123-126 | 30 |
| 26 | $OCH_3$ | $OCH_3$ | $iC_3H_7$ | H | $CH_3$ | 166-169 | 73 |
| 27 | $CH_3$ | $CH_3$ | $tC_4H_9$ | H | $CH_3$ | 178-180 | 76 |
| 28 | $CH_3$ | $CH_3$ | $tC_4H_9$ | H | 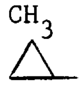 | 185-186 | 79 |
| 29 | $OCH_3$ | $CH_3$ | $tC_4H_9$ | H | $CH_3$ | 188-189 | 76 |
| 30 | $OCH_3$ | $OCH_3$ | $tC_4H_9$ | H | $CH_2CF_3$ | 159-161 | 54 |
| 31 | $OCH_3$ | $OCH_3$ | $sC_4H_9$ | H | $CH_3$ | 150-153 | 79 |
| 32 | $OCH_3$ | $OCH_3$ | $tC_4H_9$ | H | $nC_3H_7$ | 159-160 | 60 |
| 33 | $OCH_3$ | $OCH_3$ | $tC_4H_9$ | H | $iC_3H_7$ | 172-173 | 58 |
| 34 | $CH_3$ | $CH_3$ | $tC_4H_9$ | H | $nC_4H_9$ | 175-176 | 66 |
| 35 | $OCH_3$ | $OCH_3$ | $nC_4H_9$ | $CH_3$ | $CH_3$ | 122-123 | 53 |
| 36 | $OCH_3$ | $OCH_3$ | $tC_4H_9$ | H | 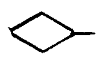 | 155-158 | 52 |
| 37 | $OCH_3$ | $OCH_3$ | $nC_4H_9$ | H | $CH_3$ | 171-174 | 79 |
| 38 | $OCH_3$ | $OCH_3$ | $tC_4H_9$ | | 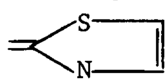 | 236-238 | 79 |
| 39 | $OCH_3$ | $CH_3$ | $tC_4H_9$ | H | 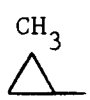 | 190-192 | 59 |
| 40 | $CH_3$ | $CH_3$ | $nC_4H_9$ | $CH_3$ | $CH_3$ | 123-124 | 50 |
| 41 | $OCH_3$ | $OCH_3$ | $sC_4H_9$ | $CH_3$ | $CH_3$ | 75-77 | 21 |
| 42 | $OCH_3$ | $OCH_3$ | $tC_4H_9$ | H | $CH_2CH{=}CH_2$ | 136-139 | 70 |
| 43 | $OCH_3$ | $OCH_3$ | $tC_4H_9$ | H | $CH_2C{\equiv}CH$ | 155-156 | 66 |
| 44 | $CH_3$ | $CH_3$ | $tC_4H_9$ | | $-(CH_2)_4-$ | 115-118 | 42 |
| 45 | $OCH_3$ | $OCH_3$ | $iC_4H_9$ | H | $CH_3$ | 156-159 | 63 |
| 46 | $CH_3$ | $CH_3$ | $iC_4H_9$ | H | $CH_3$ | 174-176 | 73 |
| 47 | $OCH_3$ | $OCH_3$ | $iC_4H_9$ | H | $nC_4H_9$ | 133-135 | 59 |
| 48 | $CH_3$ | $CH_3$ | $tC_4H_9$ | | $-(CH_2)_2O(CH_2)_2-$ | 154-156 | 76 |
| 49 | $OCH_3$ | $OCH_3$ | $tC_4H_9$ | H | $C_2H_5$ | 173-175 | 68 |

Table I (continued)

| Ex No | $R^1$ | $R^2$ | $R^3$ | $R^5$ | $R^6$ | mp (°C) | Yield (%) |
|---|---|---|---|---|---|---|---|
| 50 | $CH_3$ | $CH_3$ | $tC_4H_9$ | H | $C_2H_5$ | 176-178 | 65 |
| 51 | $OCH_3$ | $OCH_3$ | $iC_3H_7$ | H | $C_2H_5$ | 177-178 | 72 |
| 52 | $OCH_3$ | $OCH_3$ | $iC_4H_9$ | H | $C_2H_5$ | 147-149 | 74 |
| 53 | $CH_3$ | $CH_3$ | $iC_4H_9$ | H | $C_2H_5$ | 169-171 | 73 |
| 54 | $OCH_3$ | $OCH_3$ | $sC_4H_9$ | H | $C_2H_5$ | 142-144 | 77 |
| 55 | $CH_3$ | $CH_3$ | $nC_4H_9$ | H | $C_2H_5$ | 107-108 | 53 |
| 56 | $CH_3$ | $CH_3$ | $tC_4H_9$ | H | $nC_3H_7$ | 170-171 | 73 |
| 57 | $CH_3$ | $CH_3$ | $tC_4H_9$ | H | $iC_3H_7$ | 191-192 | 54 |
| 58 | $OCH_3$ | $OCH_3$ | $iC_3H_7$ | H | $nC_3H_7$ | 165-167 | 74 |
| 59 | $OCH_3$ | $OCH_3$ | $iC_3H_7$ | H | $iC_3H_7$ | 179-182 | 57 |
| 60 | $CH_3$ | $CH_3$ | $nC_3H_7$ | H | $CH_3$ | 151-154 | 58 |
| 61 | $OCH_3$ | $OCH_3$ | $tC_4H_9$ | | 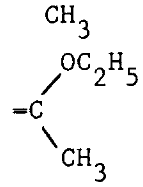 | 110-113 | 33 |
| 62 | $OCH_3$ | $OCH_3$ | $tC_4H_9$ | H | H | 176 | 63 |
| 63 | $OCH_3$ | $OCH_3$ | $nC_3H_7$ | H | $CH_3$ | 177-179 | 70 |
| 64 | $CH_3$ | $CH_3$ | $nC_3H_7$ | H | $CH_3$ | 151-154 | 58 |
| 65 | $OCH_3$ | $OCH_3$ | $nC_3H_7$ | $CH_3$ | $CH_3$ | 74-76 | 35 |
| 66 | $OCH_3$ | $OCH_3$ | $tC_4H_9$ | $CH_3$ | $C_2H_5$ | 131-134 | 48 |
| 67 | $CH_3$ | $CH_3$ | $tC_4H_9$ | $CH_3$ | $C_2H_5$ | 120-122 | 47 |
| 68 | $CH_3$ | $CH_3$ | $tC_4H_9$ | H | $CH_2CH{=}CH_2$ | 169-171 | 71 |
| 69 | $CH_3$ | $CH_3$ | $tC_4H_9$ | H | $CH_2C{\equiv}CH$ | 155-156 | 51 |
| 70 | $OCH_3$ | $OCH_3$ | $iC_3H_7$ | H | $CH_2C{\equiv}CH$ | 165-168 | 80 |
| 71 | $OCH_3$ | $OCH_3$ | $tC_4H_9$ | H | $CH_2CO_2CH_3$ | 148-151 | 61 |
| 72 | $OCH_3$ | $OCH_3$ | $sC_4H_9$ | H | $CH_2C{\equiv}CH$ | 130-134 | 67 |
| 73 | $OCH_3$ | $OCH_3$ | $tC_4H_9$ | $CH_3$ | $OCH_3$ | 112-115 | 11 |
| 74 | $OCH_3$ | $OCH_3$ | $iC_4H_9$ | H | $CH_2C{\equiv}CH$ | 154-157 | 74 |
| 75 | $OCH_3$ | $OCH_3$ | $sC_4H_9$ | H | $CH_2CH{=}CH_2$ | 131-134 | 69 |
| 76 | $OCH_3$ | $OCH_3$ | $C_6H_5$ | H | $CH_3$ | 191-193 | 69 |
| 77 | $OCH_3$ | $OCH_3$ | $C_6H_5$ | $CH_3$ | $CH_3$ | 148-151 | 54 |

Table I (continued)

| Ex No | $R^1$ | $R^2$ | $R^3$ | $R^5$ | $R^6$ | mp (°C) | Yield (%) |
|---|---|---|---|---|---|---|---|
| 78 | $OCH_3$ | $OCH_3$ | $nC_4H_9$ | H | $CH_2C{\equiv}CH$ | 145-148 | 18 |
| 79 | $CH_3$ | $CH_3$ | $tC_4H_9$ | H | H | 178-181 | 39 |
| 80 | $OCH_3$ | $OCH_3$ | $sC_4H_9$ | H | H | 161-163 | 94 |
| 81 | $OCH_3$ | $OCH_3$ | $iC_4H_9$ | H | H | 157 | 34 |
| 82 | $OCH_3$ | $OCH_3$ | $nC_3H_7$ | H | H | 156-157 | 43 |
| 83 | $CH_3$ | $CH_3$ | $iC_4H_9$ | H | H | 162-163 | 63 |
| 84 | $OCH_3$ | $OCH_3$ | $sC_4H_9$ | $CH_3$ | $C_2H_5$ | oil | 76 |
| 85 | $OCH_3$ | $OCH_3$ | $tC_4H_9$ | H | $(CH_2)_2Cl$ | 169-171 | 64 |
| 86 | $CH_3$ | $CH_3$ | $tC_4H_9$ | H | $(CH_2)_2Cl$ | 158-161 | 63 |
| 87 | $CH_3$ | $CH_3$ | $nC_4H_9$ | H | $CH_2C{\equiv}CH$ | 106-109 | 32 |
| 88 | $OCH_3$ | $OCH_3$ | $nC_4H_9$ | H | $CH_2CH{=}CH_2$ | 152-154 | 15 |
| 89 | $CH_3$ | $CH_3$ | $iC_4H_9$ | H | $CH_2CH{=}CH_2$ | 123-126 | 34 |
| 90 | $CH_3$ | $CH_3$ | $nC_4H_9$ | H | $CH_2CH{=}CH_2$ | 119-123 | 46 |
| 91 | $OCH_3$ | $OCH_3$ | $tC_4H_9$ | H | $CH_2CH(OCH_3)_2$ | 149-152 | 72 |
| 92 | $CF_3$ | $OCH_3$ | $tC_4H_9$ | H | H | 93-96 | 24 |
| 93 | $CF_3$ | $OCH_3$ | $tC_4H_9$ | H | $CH_3$ | 179-181 | 29 |
| 94 | $OCH_3$ | $OCH_3$ | $tC_4H_9$ | H | 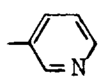 | 158-161 | 32 |
| 95 | $OCH_3$ | $OCH_3$ | $tC_4H_9$ | $CH_3$ | $CH_2C_6H_5$ | oil | 29 |
| 96 | $OCH_3$ | $OCH_3$ | $tC_4H_9$ | H | 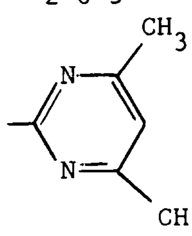 | 156 | 13 |
| 97 | $OCH_3$ | $OCH_3$ | $nC_3H_7$ | H | $CH_2CH{=}CH_2$ | 154-156 | 75 |
| 98 | $OCH_3$ | $OCH_3$ | $nC_3H_7$ | H | $CH_2C{\equiv}CH$ | 159-161 | 67 |
| 99 | $CH_3$ | $CH_3$ | $iC_4H_9$ | H | $CH_2C{\equiv}CH$ | 139-141 | 67 |
| 100 | $OCH_3$ | $OCH_3$ | $nC_3H_7$ | H | 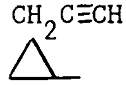 | 183-186 | 43 |
| 101 | $OCH_3$ | $OCH_3$ | $iC_4H_9$ | H | $CH_2CH{=}CH_2$ | 138-140 | 45 |
| 102 | $OCH_3$ | $OCH_3$ | $iC_4H_9$ | H | 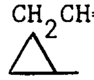 | 175-177 | 52 |

Table I (continued)

| Ex No | $R^1$ | $R^2$ | $R^3$ | $R^5$ | $R^6$ | mp (°C) | Yield (%) |
|---|---|---|---|---|---|---|---|
| 103 | $CH_3$ | $CH_3$ | $iC_4H_9$ | H | 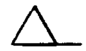 | 160-164 | 58 |
| 104 | $OCH_3$ | $OCH_3$ | $nC_4H_9$ | H | 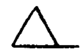 | 178-182 | 37 |
| 105 | $OCH_3$ | $OCH_3$ | $sC_4H_9$ | H | 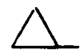 | 167-169 | 54 |
| 106 | $OCH_3$ | $OCH_3$ | $nC_3H_7$ | H | $C_2H_5$ | 160-164 | 56 |
| 107 | $OCH_3$ | $OCH_3$ | $nC_3H_7$ | H | $nC_3H_7$ | 155-158 | 37 |
| 108 | $OCH_3$ | $OCH_3$ | $nC_4H_9$ | H | $C_2H_5$ | 170-172 | 21 |
| 109 | $OCH_3$ | $OCH_3$ | $sC_4H_9$ | H | $nC_3H_7$ | 93-97 | 16 |
| 110 | $OCH_3$ | $OCH_3$ | $tC_4H_9$ | H | $OCH_3$ | 176-179 | 37 |
| 111 | $OCH_3$ | $OCH_3$ | $iC_3H_7$ | H | H | 166-169 | 93 |
| 112 | $OCH_3$ | $OCH_3$ | $iC_3H_7$ | H | 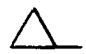 | 186-188 | 39 |
| 113 | $OCH_3$ | $OCH_3$ | $iC_3H_7$ | H | $CH_2CH{=}CH_2$ | 155-157 | 40 |
| 114 | $OCH_3$ | $OCH_3$ | $OCH_3$ | $CH_3$ | $CH_3$ | 139-143 | 15 |
| 115 | $OCH_3$ | $OCH_3$ | $nC_4H_9$ | H | H | 169-170 | 52 |
| 116 | $OCH_3$ | $OCH_3$ | $nC_4H_9$ | H | $(CH_2)_2Cl$ | 158-160 | 27 |
| 117 | $OCH_3$ | $OCH_3$ | $iC_4H_9$ | H | $(CH_2)_2Cl$ | 174-175 | 53 |
| 118 | $OCH_3$ | $OCH_3$ | $sC_4H_9$ | H | $(CH_2)_2Cl$ | 147-150 | 43 |
| 119 | $OCH_3$ | $OCH_3$ | $iC_3H_7$ | H | $(CH_2)_2Cl$ | 163-165 | 51 |
| 120 | $OCH_3$ | $OCH_3$ | $CH_3$ | H | $nC_4H_9$ | 138-141 | 40 |
| 121 | $OCH_3$ | $OCH_3$ | $CH_3$ | H | $CH_2C_6H_5$ | 183-184 | 47 |
| 122 | $OCH_3$ | $OCH_3$ | $sC_4H_9$ | H | $nC_4H_9$ | 127-129 | 40 |
| 123 | $OCH_3$ | $OCH_3$ | $sC_4H_9$ | H | $iC_3H_7$ | 156-157 | 11 |
| 124 | $OCH_3$ | $OCH_3$ | $nC_4H_9$ | H | $nC_3H_7$ | 154-155 | 31 |
| 125 | $OCH_3$ | $OCH_3$ | $tC_4H_9$ | H | $(CH_2)_2OCH_3$ | 132-135 | 68 |
| 126 | $OCH_3$ | $OCH_3$ | $CH_3$ | H | 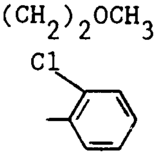 | 171-173 | 26 |
| 127 | $OCH_3$ | $OCH_3$ | $C_6H_5$ | H | H | 177-178 | 32 |
| 128 | $CH_3$ | $CH_3$ | $nC_4H_9$ | H | H | 169-170 | 25 |
| 129 | $OCH_3$ | $OCH_3$ | $tC_4H_9$ | H | 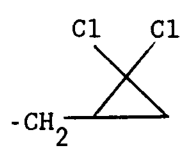 | 156-157 | 53 |

Table I (continued)

| Ex No | $R^1$ | $R^2$ | $R^3$ | $R^5$ | $R^6$ | mp (°C) | Yield (%) |
|---|---|---|---|---|---|---|---|
| 130 | $OCH_3$ | $OCH_3$ | $sC_4H_9$ | $CH_3$ | $OCH_3$ | 138-144 | 10 |
| 131 | $OCH_3$ | $OCH_3$ | $C_6H_5$ | H | $CH_2C_6H_5$ | 200-201 | 41 |
| 132 | $OCH_3$ | $OCH_3$ | $nC_4H_9$ | $CH_3$ | $C_2H_5$ | 103-104 | 4 |
| 133 | $OCH_3$ | $OCH_3$ | $sC_4H_9$ | H | $OCH_3$ | 136-137 | 33 |
| 134 | $OCH_3$ | $OCH_3$ | $C_2H_5$ | H | H | 167-168 | 77 |
| 135 | $OCH_3$ | $OCH_3$ | $C_2H_5$ | H | $CH_3$ | 171-174 | 59 |
| 136 | $OCH_3$ | $OCH_3$ | $C_2H_5$ | $CH_3$ | $CH_3$ | oil | 55 |
| 137 | $CH_3$ | $OCH_3$ | $tC_4H_9$ | H | H | 182-184 | 47 |
| 138 | $OCH_3$ | $OCH_3$ | $tC_4H_9$ | $C_2H_5$ | $C_2H_5$ | 134-142 | 39 |
| 139 | $OCH_3$ | $OCH_3$ | $tC_4H_9$ | | 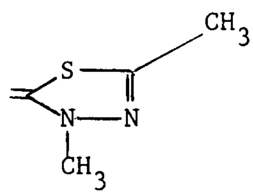 | 207-211 | 80 |
| 140 | $OCH_3$ | $OCH_3$ | $sC_4H_9$ | | 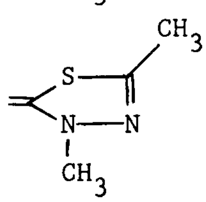 | 148-151 | 78 |
| 141 | $OCH_3$ | $OCH_3$ | $tC_4H_9$ | | 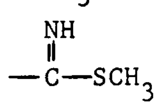 | 178-180 | 33 |
| 142 | $OCH_3$ | $OCH_3$ | $iC_3h_7$ | | 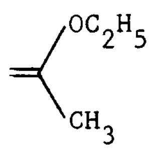 | 148-150 | 19 |
| 143 | $OCH_3$ | $OCH_3$ | $iC_3H_7$ | | 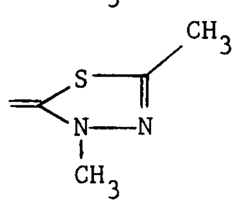 | 167-170 | 27 |
| 144 | $OCH_3$ | $OCH_3$ | $tC_4H_9$ | | 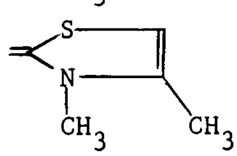 | 235-238 | 60 |

Table I (continued)

| Ex No | $R^1$ | $R^2$ | $R^3$ | $R^5$ | $R^6$ | mp (°C) | Yield (%) |
|---|---|---|---|---|---|---|---|
| 145 | $OCH_3$ | $OCH_3$ | $iC_4H_9$ | | 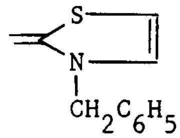 | 187-189 | 63 |
| 146 | $OCH_3$ | $OCH_3$ | $iC_4H_9$ | | 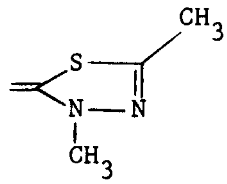 | 178-179 | 11 |
| 147 | $OCH_3$ | $OCH_3$ | $nC_4H_9$ | | 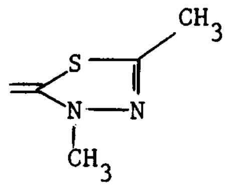 | 160-162 | 13 |
| 148 | $OCH_3$ | $OCH_3$ | $nC_3H_7$ | | 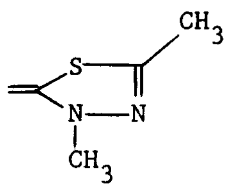 | 171-173 | 12 |
| 149 | $OCH_3$ | $OCH_3$ | $tC_4H_9$ | | 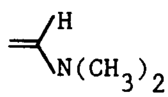 | 187-191 | 54 |
| 150 | $OCH_3$ | $OCH_3$ | $sC_4H_9$ | | 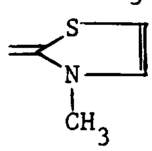 | 172-176 | 58 |
| 151 | $OCH_3$ | $OCH_3$ | $iC_4H_9$ | | 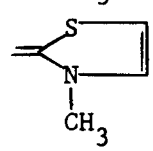 | 183-185 | 11 |
| 152 | $CH_3$ | $CH_3$ | $tC_4H_9$ | | 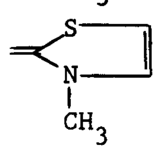 | 175-176 | 35 |
| 153 | $CH_3$ | $OCH_3$ | $tC_4H_9$ | | 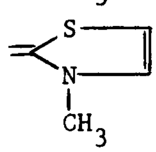 | 181-184 | 55 |
| 154 | $OCH_3$ | $OCH_3$ | $sC_4H_9$ | | 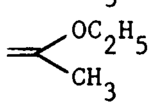 | 111-112 | 38 |

Table I (continued)

| Ex No | $R^1$ | $R^2$ | $R^3$ | $R^5$ | $R^6$ | mp (°C) | Yield (%) |
|---|---|---|---|---|---|---|---|
| 155 | $OCH_3$ | $OCH_3$ | $iC_4H_9$ | 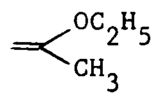 | | 126-128 | 39 |
| 156 | $OCH_3$ | $OCH_3$ | $nC_4H_9$ | 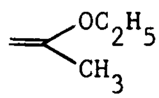 | | 129-130 | 41 |
| 157 | $CH_3$ | $CH_3$ | $tC_4H_9$ | 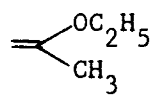 | | 151-153 | 22 |
| 158 | $CH_3$ | $OCH_3$ | $tC_4H_9$ | 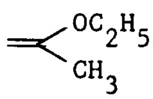 | | oil | 28 |

Table II

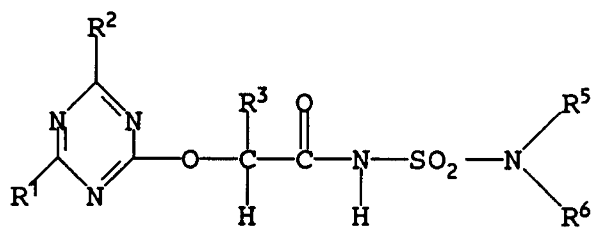

| Ex No | $R^1$ | $R^2$ | $R^3$ | $R^5$ | $R^6$ | mp (°C) | Yield (%) |
|---|---|---|---|---|---|---|---|
| 159 | $OCH_3$ | $OCH_3$ | $tC_4H_9$ | H | H | 145-148 | 62 |
| 160 | $OCH_3$ | $OCH_3$ | $tC_4H_9$ | H | $CH_3$ | 187-188 | 54 |
| 161 | $OCH_3$ | $OCH_3$ | $tC_4H_9$ | $CH_3$ | $CH_3$ | oil | 32 |
| 162 | $OCH_3$ | $OCH_3$ | $tC_4H_9$ | $CH_3$ | $C_2H_5$ | oil | 21 |
| 163 | $CH_3$ | $OCH_3$ | $tC_4H_9$ | H | $CH_3$ | 141-144 | 35 |
| 164 | $CH_3$ | $OCH_3$ | $tC_4H_9$ | H | H | 179-180 | 65 |
| 165 | $OCH_3$ | $OCH_3$ | $tC_4H_9$ | 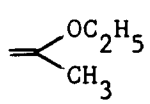 | | oil | 16 |
| 166 | $OCH_3$ | $OCH_3$ | $tC_4H_9$ | 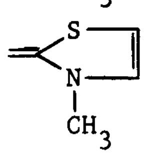 | | 200-104 | 39 |

Elemental analysis or n.m.r. data for the compounds of general formula I described above is set out in Table III below.

Table II

$^1H$ n.m.r. - chemical shift, δ, in p.p.m.

for solutions in $CDCl_3$ (unless other wise indicated)

or Analysis %

| | C | | H | | N | |
|---|---|---|---|---|---|---|
| | Calc. | Found | Calc. | Found | Calc. | Found |
| Ex No | | | | | | |
| 3 | 43.1 | 43.3 | 6.1 | 6.2 | 15.5 | 15.5 |
| 4 | 44.7 | 44.9 | 6.4 | 6.7 | 14.9 | 15.0 |
| 5 | 50.9 | 51.2 | 5.7 | 6.0 | 13.2 | 13.1 |
| 6 | 1.1(9H,s), 3.3(4H,m), 3.7(4H,m), 3.9(6H,s), 4.9(1H,s), 5.7(1H,s), 8.4(1H,br.s) | | | | | |
| 7 | 47.3 | 47.4 | 6.8 | 6.9 | 16.2 | 16.0 |
| 8 | 1.1(9H,s), 1.9(4H,m), 3.3-3.5(4H,m), 3.9(6H,s), 4.9(1H,s), 5.8(1H,s), 8.5(1H,br.s) | | | | | |
| 9 | 52.0 | 52.1 | 6.0 | 6.2 | 12.8 | 12.8 |
| 10 | 0.9(3H,t), 1.1(9H,s), 1.3(2H,m), 1.5(2H,m), 2.9(2H,q), 3.9(6H,s), 4.9(1H,s), 5.1(1H,br.t), 5.8(1H,s), 8.5(1H,br.s) | | | | | |
| 11 | 52.0 | 52.1 | 6.0 | 6.3 | 12.8 | 12.8 |
| 12 | 44.7 | 44.4 | 6.4 | 6.6 | 14.9 | 14.6 |
| 13 | 48.8 | 49.0 | 7.0 | 6.8 | 16.3 | 16.2 |
| 14 | 48.8 | 48.9 | 7.0 | 7.0 | 16.3 | 16.1 |
| 15 | 52.7 | 53.1 | 5.5 | 5.6 | 15.4 | 15.5 |
| 16 | 54.0 | 54.3 | 4.5 | 4.5 | 12.6 | 12.6 |
| 17 | 55.0 | 55.1 | 4.8 | 4.8 | 12.2 | 12.2 |
| 18 | 41.0 | 41.1 | 5.6 | 5.6 | 14.7 | 14.6 |
| 19 | 39.3 | 40.1 | 5.2 | 5.3 | 15.3 | 15.2 |
| 20 | 47.3 | 47.7 | 6.7 | 6.8 | 17.0 | 17.1 |
| 21 | 46.7 | 46.9 | 6.7 | 6.8 | 15.5 | 15.4 |
| 22 | 46.4 | 46.4 | 6.2 | 6.2 | 14.4 | 14.4 |
| 23 | 48.8 | 48.5 | 7.0 | 6.9 | 16.3 | 16.1 |
| 24 | 47.3 | 47.3 | 6.7 | 6.6 | 17.0 | 16.8 |
| 25 | 43.1 | 43.4 | 6.1 | 5.7 | 15.5 | 15.5 |
| 26 | 41.4 | 41.3 | 5.8 | 5.9 | 16.1 | 16.1 |

| | | | | | | |
|---|---|---|---|---|---|---|
| 27 | 47.3 | 47.2 | 6.7 | 6.7 | 17.0 | 16.9 |
| 28 | 50.5 | 50.7 | 6.8 | 6.7 | 15.7 | 15.8 |
| 29 | 45.1 | 45.2 | 6.4 | 6.3 | 16.2 | 16.3 |
| 30 | 39.1 | 39.2 | 4.9 | 5.0 | 13.0 | 12.9 |
| 31 | 43.1 | 43.5 | 6.1 | 6.0 | 15.5 | 15.7 |
| 32 | 46.1 | 46.1 | 6.7 | 6.7 | 14.3 | 14.5 |
| 33 | 46.1 | 45.9 | 6.7 | 6.7 | 14.3 | 14.1 |
| 34 | 51.6 | 51.6 | 7.6 | 7.3 | 15.0 | 15.1 |
| 34 | 44.7 | 44.8 | 6.4 | 6.4 | 14.9 | 15.0 |
| 36 | 1.1(9H,s), 1.5-2.3(6H,m), 3.7(1H,m), 3.9(6H,s), 5.0(1H,s), 5.4(1H,d), 5.7(1H,s), 8.5(1H,br.s) | | | | | |
| 37 | 43.1 | 43.1 | 6.1 | 6.1 | 15.5 | 15.2 |
| 38 | 43.1 | 43.4 | 5.2 | 5.2 | 15.7 | 15.8 |
| 39 | 48.4 | 48.2 | 6.5 | 6.4 | 15.0 | 14.9 |
| 40 | 48.8 | 49.1 | 7.0 | 7.0 | 16.3 | 16.4 |
| 41 | 0.8(3H,t), 1.0(3H,m), 1.2(1H,m), 1.5(1H,m), 2.0(1H,m), 2.8(2x3H,s), 3.8(6H,s), 5.1 and 5.3 (1H,2xd), 5.7(1H,s), 8.5(1H,br.s) | | | | | |
| 42 | 46.4 | 46.2 | 6.2 | 6.6 | 14.4 | 14.5 |
| 43 | 46.6 | 46.0 | 5.7 | 5.9 | 14.5 | 14.4 |
| 44 | 1.1(9H,s), 1.8(4H,m), 2.4(6H,s), 3.4(4H,m), 5.0(1H,s), 6.7(1H,s), 8.4(1H,br.s) | | | | | |
| 45 | 43.1 | 42.8 | 6.1 | 6.1 | 15.5 | 15.8 |
| 46 | 47.3 | 46.8 | 6.7 | 6.7 | 17.0 | 17.1 |
| 47 | 47.5 | 47.4 | 7.0 | 7.0 | 13.9 | 14.2 |
| 48 | 49.7 | 49.8 | 6.8 | 6.9 | 14.5 | 14.7 |
| 49 | 44.7 | 44.5 | 6.4 | 6.5 | 14.9 | 15.1 |
| 50 | 48.8 | 48.5 | 7.0 | 7.1 | 16.3 | 16.3 |
| 51 | 43.1 | 42.6 | 6.1 | 6.2 | 15.5 | 15.6 |
| 52 | 0.9(6H,dd), 1.1(3H,t), 1.7(1H,m), 1.8(2H,m), 2.9(2H,q), 3.8(6H,s), 5.2(1H,br.t), 5.3(1H,dd), 5.7(1H,s), 8.6(1H,br.s) | | | | | |
| 53 | 0.9(6H,dd), 1.1(3H,t), 1.7-1.9(2H,m), 2.4(6H,s), 3.0(2H,m), 5.1(1H,br.t), 5.5(1H,dd), 6.7(1H,s), 8.9(1H,br.s) | | | | | |

| | | | | | | |
|---|---|---|---|---|---|---|
| 54 | 1.0(3H,m), 1.1(3H,dd), 1.2(3H,dt), 1.2-1.6 (2H,m), 2.0(1H,m), 3.0(2H,m), 3.9(6H,s), 5.1(1H,br.m), 5.2 and 5.4(1H, 2xd), 5.8(1H,s), 8.6(1H,br.s) | | | | | |
| 55 | 48.8 | 48.4 | 7.0 | 7.0 | 16.3 | 16.4 |
| 56 | 0.9(3H,t), 1.1(9H,s), 1.5(2H,m), 2.5(6H,s), 2.8(2H,q), 5.1(1H,s), 5.2(1H,br.t), 6.7(1H,s), 8.5(1H,br.s) | | | | | |
| 57 | 1.0(6H,dd), 1.1(9H,s), 2.4(6H,s), 3.4(1H,m), 5.0(1H,br.d), 5.1(1H,s), 6.7(1H,s), 8.5(1H,br.s) | | | | | |
| 58 | 0.9(3H,t), 1.1(6H,dd), 1.5(2H,m), 2.3(1H,m), 2.9(2H,m), 3.9(6H,s), 5.1(1H,s), 5.2(1H,s), 5.7(1H,s), 8.5(1H,br.s) | | | | | |
| 59 | 1.1(6H,dd), 1.2(6H,dd), 2.8(1H,m), 3.5(1H,m), 3.9(6H,s), 5.0(1H,br.d), 5.2(1H,d), 5.7(1H,s), 8.6(1H,br.s) | | | | | |
| 60 | 0.9(3H,t), 1.5(2H,m), 2.0(2H,m), 2.4(6H,s), 2.7(3H,d), 5.3(1H,br.q), 5.4(1H,t), 6.7(1H,s), 9.0(1H,br.s) | | | | | |
| 61 | 1.1(9H,s), 1.2(3H,t), 2.5(3H,s), 3.9(6H,s), 4.1(2H,q), 4.8(1H,s), 5.7(1H,s), 8.8(1H,br.s) | | | | | |
| 62 | 1.1(9H,s), 3.9(6H,s), 5.0(1H,s), 5.6(2H,br.s), 5.7(1H,s), 9.0(1H,br.s) | | | | | |
| 63 | 1.0(3H,t), 1.5(2H,m), 2.0(2H,m), 2.7(3H,d), 3.9(6H,s), 5.2(1H,br.q), 5.3(1H,t), 5.7(1H,s), 8.7(1H,br.s) | | | | | |
| 64 | 0.9(3H,t), 1.5(2H,m), 2.0(2H,m), 2.4(6H,s), 2.6(3H,d), 5.4(1H,q), 5.5(1H,t), 6.8(1H,S), 9.0(1H,br s) | | | | | |
| 65 | 43.1 | 42.5 | 6.1 | 6.4 | 15.5 | 15.5 |
| 66 | 46.1 | 46.3 | 6.7 | 6.8 | 14.3 | 14.7 |
| 67 | 50.3 | 50.2 | 7.3 | 7.3 | 15.6 | 15.7 |
| 68 | 1.1(9H,s), 1.7(1H, br s), 2.4(6H,s), 3.5(2H,m), 5.2-5.0(3H,m), 5.7(1H,m), 6.8(1H,s), 8.6(1H,br s) | | | | | |
| 69 | 1.1(9H,s), 1.7(1H,br s), 2.2(1H,t), 2.4(6H,s), 3.8(2H,m), 5.5(1H,br s), 6.7(1H,s), 8.7(1H,br s) | | | | | |
| 70 | 1.0(3H,d), 1.1(3H,d), 2.2(1H,t), 2.3(1H,m), 3.8(2H,d), 3.9(6H,s), 5.2(1H,d), 5.7(1H,s) | | | | | |
| 71 | 1.1(9H,s), 3.7(3H,s), 3.8(2H,s), 3.9(6H,s), 4.9(1H,s), 5.7(1H,s) | | | | | |
| 72 | 46.6 | 47.1 | 5.7 | 5.8 | 14.5 | 14.5 |

| | | | | | | |
|---|---|---|---|---|---|---|
| 73 | 1.1(9H,s), 1.6(1H,br s), 3.0(3H,s), 3.6(3H,s), 3.9(9H,s), 4.9(1H,s), 5.7(1H,s) | | | | | |
| 74 | 46.6 | 47.0 | 5.7 | 6.1 | 14.5 | 14.7 |
| 75 | 46.4 | 46.6 | 6.2 | 6.5 | 14.4 | 14.6 |
| 76 | 47.1 | 47.3 | 4.7 | 5.0 | 14.7 | 14.9 |
| 77 | 48.5 | 48.8 | 5.1 | 5.3 | 14.1 | 14.2 |
| 78 | 0.9(3H,t), 1.3-1.6(4H,m), 1.9-2.1(2H,q), 2.2(1H,t), 3.8(2H,br d), 3.9(6H,s), 5.3(1H,dd), 5.8(1H,s) | | | | | |
| 79 | 45.6 | 45.4 | 6.4 | 6.6 | 17.7 | 17.7 |
| 80 | 41.4 | 40.6 | 5.8 | 6.1 | 16.1 | 15.7 |
| 81 | 41.4 | 41.8 | 5.8 | 6.2 | 16.1 | 16.4 |
| 82 | 39.5 | 39.4 | 5.4 | 5.6 | 16.8 | 16.8 |
| 83 | 45.6 | 45.8 | 6.4 | 6.7 | 17.7 | 17.5 |
| 84 | 0.9(3H,q), 1.0-1.8(8H,m), 2.1(1H,m), 2.9(3H,2xs), 3.3(2H,m), 3.6(1H,m), 3.8(6H,s), 5.1 and 5.2(1H, 2 x d), 5.7(1H,s), 8.5(1H,br s) | | | | | |
| 85 | 40.9 | 41.2 | 5.6 | 6.0 | 13.6 | 13.7 |
| 86 | 44.4 | 44.0 | 6.1 | 6.4 | 14.8 | 14.8 |
| 87 | 0.9(3H,t), 1.2-1.5(4H,m), 2.0(2H,br q), 2.2(1H,t), 2.4(6H,s), 3.8(2H,br m), 5.4(1H,dd), 5.6(1H,br s), 6.7(1H,s) | | | | | |
| 88 | 46.4 | 46.3 | 6.2 | 6.4 | 14.4 | 14.5 |
| 89 | 1.0(6H,dd), 1.6-2.0(3H,m), 2.4(6H,s), 3.5(2H,br s), 5.1-5.4(4H,m), 5.7(1H,m), 6.8(1H,s) | | | | | |
| 90 | 50.6 | 49.9 | 6.8 | 7.0 | 15.7 | 15.8 |
| 91 | 44.0 | 44.1 | 6.5 | 6.8 | 12.8 | 12.9 |
| 92 | 37.3 | 38.0 | 4.4 | 4.8 | 14.5 | 14.6 |
| 93 | 39.0 | 39.2 | 4.8 | 5.0 | 14.0 | 13.9 |
| 94 | 48.0 | 47.5 | 5.4 | 5.6 | 16.5 | 17.2 |
| 95 | 1.0(9H,s), 2.6(3H,s), 3.8(6H,s), 4.3(2H,s), 4.8(1H,s), 5.7(1H,s), 8.7(1H,br s) | | | | | |
| 96 | 1.0(9H,s), 2.3(6H,s), 2.4(1H,s), 3.8(6H,s), 4.7(1H,s), 5.5(1H,s), 6.4(1H,s) | | | | | |
| 97 | 1.0(3H,t), 1.5(2H,m), 2.0(2H,m), 3.6(2H,br m), 3.9(6H,s), 5.1-5.3(3H,m), 5.7(1H,m) | | | | | |

| | | | | | | |
|---|---|---|---|---|---|---|
| 98 | 44.2 | 44.9 | 5.4 | 5.3 | 15.1 | 14.6 |
| 99 | 1.0(6H,dd), 1.8-2.0(3H,m), 2.2(1H,t), 2.4(6H,s), 3.8(2H,m), 5.5(1H,dd), 5.6(1H,m), 6.7(1H,s) | | | | | |
| 100 | 44.9 | 44.6 | 5.9 | 6.1 | 15.0 | 14.7 |
| 101 | 46.4 | 46.2 | 6.2 | 6.5 | 14.4 | 14.3 |
| 102 | 46.4 | 46.1 | 6.2 | 6.3 | 14.4 | 14.5 |
| 103 | 0.6(2H,m), 0.7(2H,m), 0.9(6H,dd), 1.7-2.0(2H,m), 2.2(1H,m), 2.4(6H,s), 5.5(1H,dd), 5.7(1H,br s) | | | | | |
| 104 | 0.7(2H,m), 0.8(2H,m), 0.9(3H,t), 1.3-1.6(4H,m), 2.0(2H,q), 2.1(1H,br s), 2.3(1H,m), 3.9(6H,s), 5.3(1H,t), 5.7(1H,s), 9.1(1H,br s) | | | | | |
| 105 | 46.4 | 46.8 | 6.2 | 6.4 | 14.4 | 14.8 |
| 106 | 43.1 | 43.4 | 6.1 | 6.4 | 15.5 | 15.9 |
| 107 | 44.7 | 44.8 | 6.4 | 6.7 | 14.9 | 15.0 |
| 108 | 44.7 | 45.0 | 6.3 | 6.6 | 14.9 | 14.9 |
| 109 | 0.8-0.9(6H,2xm), 1.0(3H,2xd), 1.3-1.7(4H,m), 2.0(1H,m), 2.9(2H,q), 3.9(6H,s), 5.6(1H,m), 5.7(1H,2xd) | | | | | |
| 110 | 1.0(9H,s), 1.8(1H,br s), 3.7(3H,s), 3.8(6H,s), 4.9(1H,s), 5.7(1H,s), 8.0(1H,br s) | | | | | |
| 111 | 44.3 | 44.8 | 2.8 | 3.1 | 21.5 | 21.9 |
| 112 | 44.9 | 44.3 | 5.9 | 5.9 | 14.7 | 15.0 |
| 113 | 44.9 | 44.2 | 5.9 | 5.6 | 14.7 | 14.8 |
| 114 | 1.6(3H,d), 2.9(6H,s), 3.9(6H,s), 5.3(1H,q), 5.7(1H,s), 8.5(1H,br s) | | | | | |
| 115 | 41.4 | 41.2 | 5.8 | 5.8 | 16.1 | 15.8 |
| 116 | 40.9 | 41.1 | 5.6 | 5.8 | 13.6 | 13.6 |
| 117 | 40.9 | 40.7 | 5.6 | 5.6 | 13.6 | 13.7 |
| 118 | 40.9 | 40.7 | 5.6 | 5.7 | 13.6 | 13.7 |
| 119 | 39.4 | 40.0 | 5.3 | 5.9 | 14.1 | 14.6 |
| 120 | 43.0 | 42.6 | 6.1 | 6.8 | 15.5 | 16.1 |
| 121 | 48.5 | 48.7 | 5.1 | 5.6 | 14.1 | 14.8 |
| 122 | 47.5 | 48.0 | 7.0 | 7.6 | 13.9 | 14.7 |
| 123 | 46.1 | 45.5 | 6.1 | 6.8 | 14.4 | 14.8 |
| 124 | 46.1 | 45.8 | 6.7 | 6.7 | 14.4 | 15.0 |

| | | | | | | |
|---|---|---|---|---|---|---|
| 125 | 1.1(9H,s), 3.1(2H,br m), 3.3(3H,s), 3.4(2H,t), 3.9(6H,s), 4.9(1H,s), 5.6(1H,br s), 5.7(1H,s) | | | | | |
| 126 | 43.2 | 43.2 | 4.1 | 4.5 | 13.4 | 13.8 |
| 127 | 45.7 | 45.2 | 4.4 | 4.8 | 15.2 | 15.3 |
| 128 | 45.6 | 44.9 | 6.4 | 6.6 | 17.7 | 17.8 |
| 129 | 1.0(9H,s), 1.2(1H,d t), 1.6(1H,d d), 1.9(1H m), 3.0(1H,m), 3.3(1H,m), 3.9(6H,s), 4.9(1H,s), 5.6(1H,br m), 5.7(1H,s), 8.6(1H,br s) | | | | | |
| 130 | 0.8-1.0(6H,m), 1.2-1.7(2H,m), 2.0(1H,m), 2.6(3H,s), 3.6(3H,s), 3.9(6H,s), 4.9(1H,2xd), 5.7(1H,s) (in $d^6$-DMSO) | | | | | |
| 131 | 1.8(1H,br s), 3.9(6H,s), 4.0(1H,m), 4.1(1H dd), 5.5(1H,br t), 5.7(1H,s), 6.1(1H,s), 7.0-7.5(10H,m), 9.2(1H,br s) | | | | | |
| 132 | 46.1 | 45.8 | 6.7 | 6.9 | 14.4 | 14.4 |
| 133 | 41.3 | 41.4 | 5.9 | 5.7 | 14.8 | 14.9 |
| 134 | 37.5 | 37.2 | 5.0 | 5.6 | 17.5 | 17.4 |
| 135 | 39.5 | 39.1 | 5.4 | 5.8 | 16.8 | 16.7 |
| 136 | 1.0(3H,t), 2.0(2H,m), 2.8(6H,s), 3.3(6H,s), 5.0(1H,t), 5.6(1H,s), 8.9(1H,br s) | | | | | |
| 137 | 43.1 | 42.5 | 6.1 | 6.4 | 15.5 | 15.5 |
| 138 | 47.5 | 46.9 | 7.0 | 7.6 | 13.9 | 13.9 |
| 139 | 41.7 | 42.3 | 5.3 | 5.7 | 18.2 | 17.7 |
| 140 | 41.7 | 41.8 | 5.3 | 5.3 | 18.2 | 18.2 |
| 141 | 1.1(9H,s), 1.7(1H,br s), 2.3(3H,s), 3.9(H,s), 4.3(1H,s), 5.7(1H,s), 8.5(1H,br s) | | | | | |
| 142 | 44.6 | 45.2 | 6.0 | 6.4 | 13.9 | 14.1 |
| 143 | 40.4 | 40.8 | 5.0 | 5.2 | 18.8 | 19.1 |
| 144 | 44.4 | 44.3 | 5.5 | 5.7 | 15.2 | 15.2 |
| 145 | 50.7 | 49.9 | 5.2 | 5.0 | 13.4 | 13.4 |
| 146 | 41.8 | 41.1 | 5.3 | 5.0 | 18.3 | 18.0 |
| 147 | 41.7 | 41.1 | 5.3 | 5.3 | 18.3 | 18.2 |
| 148 | 40.4 | 40.7 | 5.0 | 5.2 | 18.8 | 18.4 |
| 149 | 1.0(9H,s), 2.8(3H,s), 3.1(3H,s), 3.9(6H,s), 3.6(1H,s), 5.8(1H,s), 8.0(1H,s), 11.6(1H,s) | | | | | |
| 150 | 50.3 | 50.2 | 7.3 | 7.3 | 15.6 | 15.7 |

| | | | | | | |
|---|---|---|---|---|---|---|
| 151 | 47.5 | 47.1 | 7.0 | 7.2 | 13.9 | 13.4 |
| 152 | 46.5 | 45.6 | 5.6 | 6.1 | 16.9 | 16.7 |
| 153 | 44.7 | 44.1 | 5.4 | 5.8 | 16.3 | 16.2 |
| 154 | 45.9 | 45.4 | 6.3 | 6.8 | 13.4 | 13.6 |
| 155 | 45.9 | 45.4 | 6.3 | 6.8 | 13.4 | 13.4 |
| 156 | 45.9 | 45.5 | 6.3 | 6.7 | 13.4 | 13.4 |
| 157 | 1.1(9H,s), 1.3(3H,t), 2.4(6H,s), 2.5(3H,s), 4.1(2H,q), 5.1(1H,2xs), 6.7(1H,s), 8.7(1H,br s) | | | | | |
| 158 | 1.0(9H,s), 1.2(3H,t), 2.2(3H,s), 2.4(3H,s), 3.7(3H,s), 4.0(2H,q), 4.8(1H,s), 6.1(1H,s), 8.9(1H,br s) | | | | | |
| 159 | 1.0(9H,s), 3.9(6H,s), 4.8(1H,s), 7.5(2H,s), 11.8(1H,s), (in $d^6$-DMSO) | | | | | |
| 160 | 1.0(9H,s), 2.5(3H,br d), 3.9(6H,s), 4.8(1H,s) 7.5(1H,br q), 11.8(1H,br s), (in $d^6$-DMSO) | | | | | |
| 161 | 1.0(9H,2), 2.8(6H,s), 4.0(9H,s), 5.0(1H,s), 8.4(1H,br s) | | | | | |
| 162 | 1.0(9H,s), 1.1(3H,t), 2.8(3H,s), 3.3(2H,q), 3.9(6H,s), 4.9(1H,s), 8.7(1H,br s) | | | | | |
| 163 | 1.1(9H,s), 2.5(3H,s), 2.7(3H,d), 4.0(3H,s), 5.0(1H,s), 5.2(1H,br q) | | | | | |
| 164 | 1.1(9H,s), 2.4(3H,s), 4.0(3H,s), 4.7(1H,s), 7.5(2H,br s), 11.8(1H,br s) (in $d^6$-DMSO) | | | | | |
| 165 | 1.0(9H,s), 1.2(3H,t), 2.4(3H,s), 3.9(6H,s), 4.0(2H,m), 4.8(1H,s), 9.5(1H,br s) | | | | | |
| 166 | 40.4 | 40.4 | 5.0 | 5.5 | 18.8 | 18.1 |

EXAMPLE 167

Herbicidal Activity

To evaluate their herbicidal activity, compounds according to the invention were tested using as representative range of plants: maize, Zea mays (Mz); rice, Oryza sativa (R); barnyard grass, Echinochloa crusgalli (BG); oat, Avena sativa (O); linseed, Linum usitatissimum (L); mustard, Sinapsis alba (M); sugar beet, Beta vulgaris (SB); and soya bean, Glycine max (S).

The tests fall into two categories, pre-emergence and post-emergence. The pre-emergence tests involved spraying a liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at

dosage levels corresponding to 5 kg or 1 kg of active material per hectare in a volume equivalent to 600 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect.

The results of the tests are set out in Table IV below, in which the compounds are identified by reference to the preceding Examples. Absence of a numeral in the Table indicates a zero rating; an asterisk indicates that no result was obtained.

TABLE IV

| Compound of | Soil drench 10 kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 3 | 7 | 7 | 7 | 6 | 7 | 8 | 7 | 8 | 5 | 7 | 7 | 8 | 7 | 7 | 9 | 9 | 7 | 8 | 8 | 8 | 7 | 8 | 8 | 8 | 8 |
| | | | | | | | | | 1 | 2 | 6 | 7 | 4 | 7 | 9 | 8 | 6 | 7 | 6 | 6 | 2 | 7 | 8 | 8 | 3 |
| 4 | 5 | 8 | 9 | 8 | 8 | 9 | 9 | 7 | 5 | 8 | 8 | 8 | 8 | 8 | 9 | 8 | 8 | 8 | 9 | 9 | 8 | 9 | 8 | 8 | 6 |
| | | | | | | | | | 1 | 5 | 5 | 8 | 7 | 8 | 8 | 8 | 8 | 3 | 6 | 8 | 4 | 8 | 8 | 8 | 4 |
| 5 | 4 | 6 | 6 | 5 | 5 | 6 | 7 | 5 | 5 | | 5 | 8 | 4 | 5 | 8 | 8 | 7 | 7 | 7 | 5 | | 2 | 7 | 7 | 6 |
| | | | | | | | | | 1 | | 2 | 2 | 1 | | 7 | 7 | 4 | 5 | 2 | | | | 5 | 5 | 4 |
| 6 | 6 | 6 | 6 | 4 | 7 | 8 | 8 | 6 | 5 | 6 | 7 | 9 | 7 | 8 | 8 | 9 | 8 | 8 | 8 | 6 | 6 | 7 | 8 | 7 | 4 |
| | | | | | | | | | 1 | 2 | 5 | 8 | 3 | 7 | 8 | 8 | 7 | 2 | 2 | 1 | | 2 | 7 | 5 | 1 |
| 7 | | | | | | | | | 5 | | | 4 | 2 | 2 | 8 | 7 | 5 | | | | | | | | |
| | | | | | | | | | 1 | | | | | | 7 | 5 | | | | | | | | | |
| 8 | 7 | 7 | 6 | 5 | 6 | 9 | 8 | 7 | 5 | 8 | 8 | 8 | 8 | 7 | 9 | 9 | 8 | 7 | 8 | 6 | 7 | 7 | 7 | 8 | 8 |
| | | | | | | | | | 1 | 2 | 3 | 7 | 3 | 7 | 8 | 8 | 8 | 3 | 6 | 2 | | 3 | 7 | 7 | 3 |
| 9 | 4 | 6 | 6 | 4 | 5 | 7 | 7 | 4 | 5 | 3 | 3 | 7 | 3 | 5 | 9 | 8 | 4 | 6 | 7 | 7 | 6 | 7 | 8 | 8 | 4 |
| | | | | | | | | | 1 | | | | | 3 | 8 | 8 | 2 | 2 | 5 | | 2 | | 7 | 8 | 3 |
| 10 | 8 | 7 | 7 | 8 | 8 | 9 | 7 | 7 | 5 | 6 | 7 | 7 | 7 | 8 | 8 | 8 | 7 | 8 | 8 | 8 | 8 | 8 | 8 | 8 | 7 |
| | | | | | | | | | 1 | 4 | 5 | 6 | 6 | 7 | 7 | 7 | 6 | 5 | 7 | 6 | 5 | 3 | 8 | 7 | |
| 11 | 2 | 4 | 3 | | 5 | 7 | 7 | 5 | 5 | 7 | 7 | 8 | 7 | 8 | 9 | 9 | 8 | 8 | 8 | 9 | 7 | 8 | 8 | 8 | 8 |
| | | | | | | | | | 1 | 2 | 6 | 8 | 5 | 8 | 8 | 8 | 7 | | 6 | 5 | 3 | 4 | 7 | 5 | |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 12 | 4 | 6 | 6 | 4 | 5 | 8 | 8 | 5 | 5 | 7 | 8 | 8 | 5 | 8 | 9 | 9 | 8 | 8 | 8 | 8 | 6 | 8 | 8 | 8 | 8 |
| | | | | | | | | | 1 | | 6 | 8 | 2 | 7 | 8 | 8 | 7 | 2 | 4 | 5 | 3 | 6 | 8 | 8 | 2 |
| 13 | 8 | 6 | 8 | 7 | 8 | 9 | 9 | * | 5 | 9 | 8 | 9 | 7 | 8 | 9 | 9 | 8 | 9 | 9 | 9 | 8 | 8 | 8 | 8 | 8 |
| | | | | | | | | | 1 | 7 | 7 | 9 | 5 | 8 | 9 | 8 | 7 | 6 | 6 | 7 | 4 | 7 | 8 | 8 | 6 |
| 14 | 8 | 7 | 8 | 7 | 6 | 9 | 9 | 7 | 5 | 7 | 7 | 8 | 7 | 7 | 9 | 9 | 8 | 8 | 9 | 9 | 7 | 7 | 8 | 9 | 7 |
| | | | | | | | | | 1 | 4 | 6 | 7 | 6 | 3 | 8 | 8 | 6 | 5 | 7 | 6 | 5 | 5 | 8 | 8 | |
| 15 | 6 | 6 | 7 | 6 | 4 | 9 | 9 | | 5 | 5 | 5 | 7 | 6 | 7 | 9 | 9 | 2 | 6 | 8 | 6 | 6 | 1 | 7 | 8 | |
| | | | | | | | | | 1 | 1 | 4 | 6 | 2 | 2 | 8 | 8 | | 2 | 5 | | 2 | | 7 | 7 | |
| 16 | | | 4 | | 4 | 6 | 3 | 2 | 5 | | | | | | 8 | 6 | 7 | 3 | 2 | 2 | 2 | 4 | 7 | 7 | 4 |
| | | | | | | | | | 1 | | | | | | 7 | 6 | 2 | | | 2 | 2 | 2 | 6 | 6 | |
| 17 | 3 | 2 | 5 | 2 | 5 | 6 | | 2 | 5 | 3 | | 2 | | 5 | 9 | 8 | 7 | 2 | | 2 | | | 6 | 7 | 4 |
| | | | | | | | | | 1 | | | | | | 7 | 6 | 2 | | | | | | 6 | 6 | |
| 18 | 6 | 3 | 2 | 1 | 4 | 7 | 4 | 3 | 5 | 4 | 4 | 7 | 3 | 6 | 9 | 8 | 6 | 5 | 7 | 7 | 3 | 6 | 7 | 8 | 6 |
| | | | | | | | | | 1 | 4 | | 6 | 2 | 4 | 8 | 6 | 5 | | | | | | 7 | 7 | 2 |
| 19 | 6 | 7 | * | 3 | 2 | 7 | 7 | 1 | 5 | 6 | 6 | 7 | 2 | 3 | 7 | 8 | 6 | 6 | 8 | 7 | 3 | 2 | 7 | 7 | 3 |
| | | | | | | | | | 1 | 1 | 6 | 6 | | 2 | 7 | 7 | 6 | | | | | | 7 | 6 | |
| 20 | 8 | 7 | 7 | 7 | 2 | 8 | 7 | * | 5 | 8 | 6 | 7 | 7 | 5 | 9 | 9 | 8 | 8 | 7 | 8 | 6 | 3 | 7 | 8 | 5 |
| | | | | | | | | | 1 | 5 | 6 | 6 | 5 | 4 | 8 | 7 | 6 | 6 | 6 | 6 | 5 | | 7 | 7 | 5 |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 21 | 8 | 8 | 9 | 6 | 7 | 8 | * | 4 | 5 | 8 | 7 | 9 | 7 | 8 | 9 | 9 | 9 | 9 | 8 | 9 | 7 | 8 | 8 | 8 | 7 |
| | | | | | | | | | 1 | 7 | 7 | 8 | 6 | 7 | 8 | 8 | 7 | 8 | 7 | 8 | 5 | 7 | 8 | 8 | 6 |
| 22 | 6 | 6 | 7 | 4 | 7 | 8 | 6 | 6 | 5 | 7 | 7 | 8 | 7 | 7 | 9 | 8 | 8 | 8 | 9 | 9 | 8 | 8 | 8 | 8 | 7 |
| | | | | | | | | | 1 | 6 | 7 | 8 | 6 | 7 | 8 | 8 | 7 | 6 | 7 | 7 | 5 | 6 | 8 | 8 | 6 |
| 23 | | | 5 | | | 6 | * | | 5 | 2 | | 8 | | 4 | 8 | 8 | 4 | 3 | | 7 | | | 7 | 7 | 4 |
| | | | | | | | | | 1 | | | 5 | | | 7 | 7 | 2 | | | | | | 7 | 4 | 4 |
| 24 | 4 | 5 | 6 | | | 6 | 5 | | 5 | 5 | 2 | 7 | | 5 | 8 | 8 | 6 | 7 | 5 | 7 | 3 | | 7 | 4 | 3 |
| | | | | | | | | | 1 | 2 | | 5 | | | 6 | 6 | 5 | 2 | | | 2 | | 6 | | |
| 25 | 8 | 7 | 8 | 7 | 7 | 8 | 7 | 6 | 5 | 8 | 7 | 9 | 8 | 7 | 9 | 8 | 7 | 8 | 9 | 9 | 7 | 8 | 7 | 8 | 6 |
| | | | | | | | | | 1 | 5 | 7 | 8 | 7 | 7 | 8 | 8 | 5 | 6 | 6 | 7 | 4 | 6 | 7 | 7 | 4 |
| 26 | 7 | 8 | 8 | 7 | 7 | 8 | 8 | 7 | 5 | 8 | 7 | 8 | 7 | 7 | 9 | 8 | 8 | 8 | 9 | 9 | 7 | 7 | 7 | 8 | 7 |
| | | | | | | | | | 1 | 7 | 7 | 8 | 7 | 7 | 8 | 7 | 6 | 6 | 8 | 8 | 7 | 6 | 7 | 7 | 6 |
| 27 | 8 | 8 | 8 | 7 | 6 | 7 | 8 | 7 | 5 | 8 | 7 | 9 | 7 | 8 | 9 | 9 | 8 | 9 | 9 | 9 | 7 | 7 | 7 | 8 | 9 |
| | | | | | | | | | 1 | 8 | 7 | 8 | 6 | 8 | 9 | 8 | 7 | 9 | 8 | 8 | 5 | 6 | 7 | 7 | 8 |
| 28 | 8 | 5 | 6 | 5 | 6 | 7 | 5 | 5 | 5 | 6 | 4 | 8 | 5 | 7 | 9 | 8 | 8 | 6 | 7 | 8 | 3 | 6 | 7 | 6 | 5 |
| | | | | | | | | | 1 | 5 | | 7 | 5 | 7 | 8 | 7 | 6 | 2 | 2 | 4 | 1 | 4 | 7 | 5 | 2 |
| 29 | 8 | 8 | 9 | 7 | 7 | 8 | 8 | 8 | 5 | 8 | 7 | 8 | 4 | 7 | 8 | 8 | 7 | 9 | 9 | 9 | 7 | 7 | 7 | 8 | 9 |
| | | | | | | | | | 1 | 7 | 5 | 8 | 4 | 7 | 8 | 7 | 7 | 8 | 8 | 9 | 5 | 5 | 7 | 8 | 4 |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 30 | 6 | 7 | 6 | 5 | 7 | 7 | 7 | 4 | 5 | 5 | 7 | 8 | 6 | 6 | 8 | 8 | 7 | 7 | 8 | 7 | 7 | 7 | 7 | 7 | 4 |
| | | | | | | | | | 1 | 2 | 6 | 7 | 6 | 6 | 7 | 7 | 4 | 4 | 4 | 2 | 4 | 5 | 5 | 6 | 2 |
| 31 | 8 | 8 | 8 | 7 | 7 | 9 | 9 | 7 | 5 | 7 | 7 | 9 | 7 | 7 | 8 | 8 | 8 | 8 | 9 | 9 | 8 | 7 | 7 | 8 | 7 |
| | | | | | | | | | 1 | 6 | 7 | 8 | 7 | 7 | 7 | 7 | 7 | 7 | 8 | 8 | 7 | 6 | 7 | 7 | 6 |
| 32 | 6 | 8 | 7 | 4 | 7 | 7 | 7 | 6 | 5 | 7 | 8 | 8 | 7 | 7 | 8 | 9 | 7 | 8 | 9 | 9 | 8 | 8 | 7 | 8 | 8 |
| | | | | | | | | | 1 | 6 | 7 | 7 | 6 | 7 | 7 | 7 | 2 | 6 | 8 | 7 | 2 | 7 | 7 | 7 | 5 |
| 33 | 7 | 7 | 7 | 6 | 7 | 7 | 8 | 5 | 5 | 6 | 8 | 8 | 7 | 7 | 8 | 8 | 6 | 8 | 9 | 8 | 7 | 8 | 7 | 8 | 7 |
| | | | | | | | | | 1 | 5 | 8 | 8 | 6 | 7 | 7 | 7 | 3 | 6 | 8 | 6 | 2 | 7 | 7 | 8 | 4 |
| 34 | 5 | | 6 | 1 | 2 | 7 | 2 | 2 | 5 | 4 | 2 | 7 | 3 | 6 | 7 | 7 | | 7 | 6 | 7 | 4 | 6 | 7 | 7 | 6 |
| | | | | | | | | | 1 | 2 | | 3 | | 5 | 7 | 6 | | 2 | | 2 | | | 6 | 5 | |
| 35 | 6 | 8 | 8 | 6 | 4 | 7 | 6 | 4 | 5 | 6 | 7 | 7 | 6 | 6 | 9 | 9 | 5 | 7 | 9 | 8 | 8 | 6 | 7 | 8 | 8 |
| | | | | | | | | | 1 | 4 | 7 | 7 | 5 | 5 | 8 | 8 | 4 | 4 | 8 | 7 | 5 | | 7 | 7 | 7 |
| 36 | 6 | 5 | 8 | 2 | 7 | 9 | 8 | 5 | 5 | 6 | 8 | 8 | 7 | 7 | 8 | 8 | 8 | 8 | 9 | 8 | 7 | 8 | 7 | 8 | 8 |
| | | | | | | | | | 1 | 5 | 7 | 7 | 2 | 7 | 7 | 7 | 2 | 2 | 2 | 7 | 2 | 7 | 7 | 7 | 2 |
| 37 | 7 | 7 | 8 | 7 | 3 | 7 | 7 | 7 | 5 | 7 | 7 | 7 | 5 | 6 | 8 | 8 | 7 | 7 | 9 | 8 | 7 | 4 | 7 | 7 | 7 |
| | | | | | | | | | 1 | 5 | 7 | 7 | 5 | 5 | 7 | 7 | 2 | 5 | 6 | 7 | 2 | | 7 | 7 | 2 |
| 38 | 7 | 7 | 7 | 7 | 7 | 7 | 7 | 8 | 5 | 6 | 6 | 8 | 6 | 7 | 8 | 7 | 8 | 8 | 9 | 8 | 7 | 6 | 7 | 7 | 8 |
| | | | | | | | | | 1 | 5 | 5 | 7 | 4 | 7 | 7 | 7 | 7 | 6 | 2 | 6 | 4 | 6 | 7 | 7 | 8 |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 39 | 7 | 7 | 8 | 7 | 7 | 7 | 6 | 5 | 5 | 7 | 3 | 8 | 3 | 7 | 7 | 7 | 4 | 8 | 8 | 8 | 3 | 4 | 7 | 7 | 6 |
| | | | | | | | | | 1 | 2 | 1 | 7 | 1 | 6 | 7 | 6 | 2 | 5 | 2 | 5 | 1 | | 6 | 6 | 5 |
| 40 | 5 | 6 | 6 | 7 | | 7 | 8 | | 5 | 6 | 6 | 7 | 4 | 6 | 8 | 9 | 6 | 7 | 9 | 8 | 7 | 6 | 7 | 7 | 8 |
| | | | | | | | | | 1 | 3 | 2 | 6 | 2 | 3 | 7 | 8 | 2 | 5 | 7 | 7 | 5 | 4 | 7 | 7 | 6 |
| 41 | 7 | 8 | 7 | 7 | 6 | 7 | 7 | 8 | 5 | 7 | 8 | 8 | 7 | 7 | 8 | 9 | 7 | 8 | 9 | 9 | 8 | 8 | 7 | 7 | 8 |
| | | | | | | | | | 1 | 6 | 7 | 7 | 7 | 7 | 7 | 9 | 6 | 7 | 9 | 7 | 7 | 7 | 7 | 7 | 6 |
| 42 | 6 | 8 | 7 | 7 | 7 | 7 | 7 | 6 | 5 | 6 | 7 | 7 | 7 | 7 | 8 | 7 | 7 | 8 | 9 | 8 | 7 | 8 | 7 | 8 | 8 |
| | | | | | | | | | 1 | 4 | 2 | 4 | 5 | 7 | 7 | 7 | 6 | 7 | 7 | 7 | 6 | 7 | 7 | 7 | 6 |
| 43 | 7 | 7 | 7 | 7 | 7 | 7 | 7 | 7 | 5 | 8 | 8 | 8 | 7 | 7 | 8 | 9 | 7 | 8 | 9 | 8 | 7 | 8 | 7 | 8 | 9 |
| | | | | | | | | | 1 | 7 | 7 | 7 | 6 | 7 | 8 | 8 | 6 | 7 | 7 | 7 | 6 | 7 | 7 | 7 | 8 |
| 44 | 4 | 2 | 6 | 2 | 3 | 8 | 2 | 6 | 5 | 4 | 3 | 7 | 2 | 7 | 8 | 7 | 7 | 4 | 7 | 7 | 4 | 6 | 7 | 7 | 6 |
| | | | | | | | | | 1 | 2 | 1 | 5 | | 6 | 7 | 6 | 4 | 2 | | 5 | 2 | 3 | 7 | 7 | 2 |
| 45 | 5 | 7 | 7 | 5 | 5 | 7 | 7 | 6 | 5 | 6 | 7 | 8 | 4 | 7 | 8 | 7 | 7 | 7 | 8 | 8 | 7 | 7 | 7 | 7 | 9 |
| | | | | | | | | | 1 | 4 | 2 | 7 | 2 | 4 | 7 | 7 | 5 | 5 | 6 | 7 | 4 | 2 | 7 | 7 | 6 |
| 46 | 6 | 7 | 7 | 7 | | 9 | 7 | | 5 | 7 | 7 | 7 | 7 | 6 | 8 | 8 | 6 | 8 | 8 | 8 | 7 | 6 | 7 | 7 | 6 |
| | | | | | | | | | 1 | 2 | 6 | 7 | 6 | 2 | 7 | 7 | 5 | 7 | 7 | 6 | 4 | | 7 | 7 | 5 |
| 47 | 5 | 7 | 6 | 2 | | 7 | 6 | 6 | 5 | 4 | | 6 | | 5 | 8 | 8 | 5 | 7 | 7 | 7 | 7 | | 7 | 7 | 8 |
| | | | | | | | | | 1 | | | 2 | | | 7 | 7 | 4 | 4 | | 5 | 2 | | 6 | 7 | 7 |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 48 | | | 4 | | 3 | 7 | | | 5 | 4 | | 7 | 2 | 7 | 8 | 8 | 5 | 5 | | 6 | | | 7 | | |
| | | | | | | | | | 1 | 2 | | 3 | | 6 | 7 | 7 | | 2 | | | | | 6 | | |
| 49 | 8 | 8 | 8 | 8 | 7 | 8 | 8 | 7 | 5 | 6 | 8 | 8 | 7 | 7 | 8 | 8 | 7 | 9 | 9 | 9 | 8 | 9 | 8 | 8 | 8 |
| | | | | | | | | | 1 | 3 | 7 | 8 | 6 | 7 | 7 | 7 | 5 | 7 | 8 | 8 | 7 | 8 | 8 | 8 | 7 |
| 50 | * | * | * | * | * | * | * | * | 5 | 7 | 7 | 8 | 6 | 7 | 8 | 8 | 7 | 8 | 8 | 9 | 8 | 8 | 8 | 8 | 7 |
| | | | | | | | | | 1 | 4 | 3 | 8 | 4 | 7 | 7 | 8 | 5 | 8 | 6 | 8 | 6 | 6 | 8 | 8 | 5 |
| 51 | 8 | 8 | 8 | 7 | 6 | 8 | 8 | 7 | 5 | 8 | 8 | 8 | 7 | 7 | 8 | 8 | 7 | 9 | 9 | 9 | 8 | 7 | 8 | 8 | 8 |
| | | | | | | | | | 1 | 6 | 8 | 8 | 6 | 7 | 7 | 7 | 5 | 5 | 9 | 7 | 5 | 4 | 8 | 8 | 3 |
| 52 | 7 | 8 | 7 | 4 | 2 | 8 | 8 | 5 | 5 | 5 | 6 | 8 | 2 | 5 | 8 | 8 | 5 | 8 | 8 | 9 | 6 | 7 | 7 | 8 | 8 |
| | | | | | | | | | 1 | 3 | 5 | 8 | | 3 | 6 | 7 | 2 | 3 | 3 | 6 | 5 | 2 | 7 | 8 | 5 |
| 53 | 8 | 7 | 8 | 7 | | 8 | 8 | 6 | 5 | 6 | 6 | 8 | 5 | 3 | 8 | 8 | 5 | 8 | 8 | 9 | 8 | 5 | 8 | 8 | 6 |
| | | | | | | | | | 1 | 4 | 2 | 8 | 2 | | 7 | 7 | 2 | 6 | 7 | 8 | 7 | 2 | 8 | 8 | 5 |
| 54 | 8 | 8 | 8 | 8 | 6 | 8 | 8 | 6 | 5 | 7 | 8 | 8 | 8 | 7 | 8 | 8 | 6 | 9 | 9 | 9 | 8 | 8 | 8 | 8 | 8 |
| | | | | | | | | | 1 | 4 | 7 | 8 | 7 | 7 | 7 | 7 | 4 | 6 | 9 | 7 | 7 | 5 | 8 | 8 | 6 |
| 55 | 8 | 7 | 7 | 7 | | 8 | 8 | | 5 | 5 | 4 | 8 | | 2 | 8 | 8 | 5 | 7 | 8 | 9 | 7 | | 8 | 8 | 5 |
| | | | | | | | | | 1 | 1 | 2 | 7 | | | 7 | 7 | 2 | 3 | 7 | 7 | 4 | | 8 | 7 | |
| 56 | 5 | | 7 | 6 | 5 | 7 | 6 | 5 | 5 | 7 | 2 | 8 | 5 | 7 | 8 | 8 | 5 | 8 | 6 | 8 | 7 | 7 | 7 | 7 | 7 |
| | | | | | | | | | 1 | 5 | 1 | 7 | 4 | 7 | 7 | 7 | 2 | 7 | 2 | 7 | 2 | 5 | 7 | 7 | 5 |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 57 | 4 | | 7 | | 6 | 7 | 6 | | 5 | 6 | | 7 | 4 | 7 | 7 | 7 | 3 | 7 | 7 | 7 | 4 | 7 | 7 | 7 | 4 |
| | | | | | | | | | 1 | 4 | | 5 | 2 | 6 | 7 | 7 | 2 | 5 | 2 | 6 | 2 | 6 | 7 | 7 | |
| 58 | 6 | 7 | 7 | 6 | 5 | 7 | 6 | 7 | 5 | 6 | 7 | 7 | 6 | 6 | 7 | 7 | 6 | 8 | 9 | 8 | 7 | 7 | 7 | 7 | 7 |
| | | | | | | | | | 1 | 4 | 6 | 7 | 5 | 6 | 6 | 7 | 2 | 6 | 8 | 7 | 6 | 2 | 7 | 7 | 5 |
| 59 | 6 | 7 | 7 | 6 | 4 | 7 | 4 | 5 | 5 | 6 | 7 | 7 | 5 | 7 | 7 | 7 | 4 | 8 | 9 | 8 | 7 | 7 | 7 | 7 | 7 |
| | | | | | | | | | 1 | 2 | 6 | 7 | 2 | 6 | 7 | 7 | 2 | 4 | 7 | 7 | 6 | | 6 | 7 | 2 |
| 60 | 7 | 6 | 7 | 7 | | 7 | 7 | | 5 | 5 | 4 | 7 | 4 | 7 | 8 | 8 | 4 | 7 | 7 | 7 | 7 | 6 | 7 | 7 | 4 |
| | | | | | | | | | 1 | 2 | 2 | 7 | 2 | | 7 | 7 | 2 | 6 | 5 | 5 | 2 | | 7 | 7 | 2 |
| 61 | 7 | 8 | 8 | 6 | 7 | 8 | 8 | 7 | 5 | 6 | 7 | 8 | 6 | 7 | 8 | 8 | 9 | 8 | 9 | 9 | 7 | 8 | 7 | 7 | 8 |
| | | | | | | | | | 1 | 4 | 5 | 8 | 2 | 7 | 7 | 7 | 8 | 7 | 7 | 7 | 4 | 7 | 7 | 7 | 8 |
| 62 | 7 | 8 | 8 | 7 | 7 | 7 | 7 | 8 | 5 | 7 | 7 | 7 | 7 | 7 | 7 | 8 | 8 | 9 | 9 | 9 | 8 | 8 | 7 | 7 | 8 |
| | | | | | | | | | 1 | 6 | 7 | 7 | 6 | 7 | 7 | 7 | 7 | 8 | 9 | 8 | 6 | 7 | 7 | 7 | 8 |
| 63 | 7 | 8 | 8 | 7 | 5 | 7 | 7 | 6 | 5 | 8 | 8 | 7 | 5 | 7 | 8 | 8 | 7 | 8 | 9 | 8 | 7 | 7 | 7 | 7 | 8 |
| | | | | | | | | | 1 | 7 | 7 | 7 | 2 | 7 | 7 | 7 | 6 | 6 | 9 | 7 | 7 | 7 | 7 | 7 | 6 |
| 64 | 7 | 6 | 7 | 7 | | 7 | 7 | | 5 | 5 | 4 | 7 | 4 | 7 | 8 | 8 | 4 | 7 | 7 | 7 | 7 | 6 | 7 | 7 | 4 |
| | | | | | | | | | 1 | 2 | 2 | 7 | 2 | | 7 | 7 | 2 | 6 | 5 | 5 | 2 | | 7 | 7 | 2 |
| 65 | 7 | 8 | 7 | 7 | 7 | 7 | 6 | 7 | 5 | 8 | 8 | 8 | 7 | 7 | 8 | 8 | 8 | 8 | 9 | 8 | 8 | 8 | 7 | 7 | 8 |
| | | | | | | | | | 1 | 5 | 8 | 7 | 6 | 7 | 7 | 7 | 8 | 6 | 9 | 7 | 7 | 6 | 7 | 6 | |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 66 | 7 | 8 | 8 | 7 | 7 | 7 | 7 | 7 | 5 | 7 | 8 | 8 | 7 | 7 | 8 | 8 | 8 | 8 | 9 | 9 | 8 | 8 | 8 | 8 | 9 |
| | | | | | | | | | 1 | 5 | 8 | 7 | 6 | 7 | 7 | 7 | 7 | 7 | 8 | 8 | 2 | 7 | 7 | 7 | 2 |
| 67 | 7 | 6 | 7 | 7 | 7 | 7 | 7 | 7 | 5 | 8 | 7 | 9 | 7 | 7 | 8 | 8 | 8 | 9 | 9 | 9 | 7 | 8 | 7 | 8 | 8 |
| | | | | | | | | | 1 | 7 | 2 | 8 | 5 | 7 | 7 | 7 | 7 | 8 | 4 | 8 | 2 | 7 | 7 | 7 | 2 |
| 68 | 6 | 6 | 5 | 6 | 6 | 7 | 6 | 5 | 5 | 7 | 7 | 8 | 6 | 7 | 8 | 9 | 7 | 8 | 8 | 7 | 7 | 7 | 7 | 7 | 7 |
| | | | | | | | | | 1 | 5 | | 7 | 4 | 7 | 8 | 7 | 5 | 7 | 6 | 6 | 5 | | 7 | 6 | 4 |
| 69 | 7 | 7 | 7 | 6 | 5 | 7 | 7 | 6 | 5 | 6 | 7 | 8 | 6 | 7 | 8 | 8 | 8 | 7 | 9 | 8 | 6 | 7 | 7 | 7 | 8 |
| | | | | | | | | | 1 | 4 | 2 | 7 | 4 | 7 | 8 | 7 | 6 | 6 | 6 | 6 | 5 | | 7 | 7 | 2 |
| 70 | 7 | 8 | 7 | 6 | 6 | 7 | 8 | 6 | 5 | 7 | 7 | 8 | 7 | 7 | 7 | 8 | 7 | 8 | 9 | 8 | 7 | 7 | 7 | 8 | 8 |
| | | | | | | | | | 1 | 5 | 7 | 7 | 7 | 7 | 7 | 7 | 6 | 6 | 8 | 7 | 5 | 2 | 7 | 7 | 7 |
| 71 | 5 | 8 | 7 | 2 | 2 | 7 | 4 | 2 | 5 | 3 | 7 | 8 | 6 | 7 | 7 | 7 | 5 | 5 | 9 | 8 | 4 | 6 | 7 | 8 | 4 |
| | | | | | | | | | 1 | | 2 | 7 | 5 | 6 | 7 | 7 | 2 | 2 | 6 | 6 | 2 | 2 | 7 | 7 | 2 |
| 72 | 7 | 8 | 7 | 7 | 6 | 7 | 7 | 4 | 5 | 7 | 8 | 8 | 7 | 7 | 7 | 8 | 6 | 8 | 9 | 9 | 6 | 7 | 7 | 8 | 7 |
| | | | | | | | | | 1 | 4 | 8 | 8 | 5 | 6 | 7 | 7 | 6 | 7 | 9 | 8 | 5 | 6 | 7 | 7 | 7 |
| 73 | 6 | 7 | 7 | 4 | 7 | 7 | 7 | 5 | 5 | 5 | 7 | 7 | 4 | 7 | 7 | 7 | 6 | 6 | 9 | 4 | 5 | 8 | 7 | 4 | 5 |
| | | | | | | | | | 1 | * | * | * | * | * | * | * | * | * | * | * | * | * | * | * | * |
| 74 | 8 | 4 | 8 | 7 | 4 | 7 | 7 | 5 | 5 | 8 | 5 | 8 | 4 | 7 | 7 | 8 | 7 | 8 | 8 | 8 | 8 | 7 | 7 | 8 | 7 |
| | | | | | | | | | 1 | 6 | 4 | 8 | 2 | 6 | 7 | 7 | 6 | 6 | 7 | 7 | 6 | 4 | 7 | 7 | 6 |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 75 | 7 | 7 | 8 | 7 | 4 | 7 | 7 | 4 | 5 | 8 | 6 | 8 | 7 | 7 | 8 | 8 | 7 | 8 | 9 | 8 | 8 | 4 | 7 | 8 | 5 |
| | | | | | | | | | 1 | 6 | 5 | 8 | 6 | 6 | 7 | 7 | 5 | 6 | 8 | 7 | 6 | | 7 | 8 | 4 |
| 76 | 5 | 2 | 7 | 6 | 4 | 7 | 8 | 5 | 5 | 6 | | 8 | 1 | 6 | 8 | 8 | 4 | 7 | 7 | 8 | 4 | 2 | 7 | 7 | 7 |
| | | | | | | | | | 1 | 2 | | 7 | | 4 | 7 | 7 | 2 | 5 | 5 | 7 | 2 | | 7 | 7 | 4 |
| 77 | 5 | 2 | 7 | 2 | 3 | 6 | 7 | 4 | 5 | 6 | 1 | 8 | 4 | 4 | 8 | 8 | 4 | 6 | 7 | 7 | 2 | 2 | 7 | 8 | 6 |
| | | | | | | | | | 1 | 2 | | 7 | | 2 | 7 | 7 | 2 | 5 | 4 | 6 | | | 7 | 7 | 2 |
| 78 | 7 | 6 | 7 | 6 | | 7 | 7 | 6 | 5 | 5 | 6 | 7 | 5 | 6 | 7 | 7 | 2 | 7 | 8 | 7 | 5 | | 7 | 8 | 6 |
| | | | | | | | | | 1 | 2 | 6 | 6 | 2 | 2 | 7 | 7 | | 6 | 7 | 7 | 2 | | 7 | 8 | 2 |
| 79 | 7 | 6 | 7 | 6 | 7 | 8 | 7 | 7 | 5 | 6 | 4 | 7 | 4 | 7 | 9 | 9 | 6 | 8 | 9 | 8 | 7 | 7 | 8 | 8 | 8 |
| | | | | | | | | | 1 | 4 | 2 | 7 | 2 | 7 | 8 | 9 | 5 | 7 | 8 | 6 | 4 | 2 | 7 | 7 | 8 |
| 80 | 7 | 6 | 7 | 4 | 5 | 7 | 7 | 7 | 5 | 7 | 6 | 7 | 6 | 7 | 7 | 8 | 6 | 7 | 9 | 8 | 6 | 4 | 7 | 8 | 8 |
| | | | | | | | | | 1 | 6 | 5 | 7 | 5 | 6 | 7 | 7 | 4 | 5 | 9 | 7 | 2 | | 7 | 8 | 8 |
| 81 | 6 | 5 | 7 | 2 | | 7 | 7 | 6 | 5 | 6 | 4 | 8 | 4 | 5 | 7 | 8 | 6 | 7 | 7 | 8 | 6 | 5 | 7 | 7 | 8 |
| | | | | | | | | | 1 | 2 | 4 | 7 | 2 | 2 | 7 | 7 | 2 | 5 | 2 | 7 | | | 7 | 7 | 6 |
| 82 | 7 | 6 | 7 | 6 | 5 | 7 | 7 | 6 | 5 | 6 | 5 | 7 | 4 | 7 | 9 | 9 | 5 | 7 | 9 | 7 | 4 | | 7 | 8 | 8 |
| | | | | | | | | | 1 | 4 | 5 | 6 | 2 | 6 | 8 | 8 | 2 | 5 | 6 | 4 | 2 | | 7 | 8 | 7 |
| 83 | 7 | 5 | 6 | 7 | | 9 | 7 | 6 | 5 | 7 | 4 | 7 | 4 | 5 | 9 | 8 | 6 | 8 | 9 | 9 | 7 | | 7 | 8 | 7 |
| | | | | | | | | | 1 | 5 | 2 | 6 | 2 | | 8 | 8 | 5 | 7 | 8 | 7 | 6 | | 7 | 7 | 6 |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 84 | 8 | 6 | 8 | 8 | 7 | 6 | 6 | 7 | 5 | 8 | 6 | 8 | 8 | 7 | 8 | 8 | 8 | 8 | 9 | 8 | 8 | 8 | 8 | 8 | 6 |
| | | | | | | | | | 1 | 7 | 6 | 8 | 7 | 6 | 8 | 8 | 7 | 7 | 9 | 8 | 7 | 6 | 8 | 7 | 2 |
| 85 | 8 | 6 | 8 | 8 | 7 | 8 | 8 | 6 | 5 | 7 | 6 | 8 | 7 | 7 | 8 | 8 | 8 | 8 | 9 | 8 | 8 | 8 | 8 | 8 | 7 |
| | | | | | | | | | 1 | 6 | 6 | 7 | 5 | 6 | 7 | 7 | 8 | 7 | 7 | 8 | 6 | 6 | 7 | 7 | 5 |
| 86 | 7 | 2 | 7 | 4 | 6 | 7 | 6 | 6 | 5 | 5 | 5 | 7 | 2 | 6 | 8 | 7 | 7 | 7 | 8 | 8 | 7 | 5 | 8 | 7 | 1 |
| | | | | | | | | | 1 | 2 | 2 | 6 | | 5 | 7 | 6 | 6 | 2 | 1 | 2 | 1 | 1 | 7 | 5 | 1 |
| 87 | 3 | 4 | 2 | 3 | | 7 | 6 | | 5 | 4 | 3 | 7 | 4 | | 7 | 8 | 4 | 7 | 7 | 8 | 6 | 4 | 7 | 7 | |
| | | | | | | | | | 1 | 2 | | 5 | 2 | | 7 | 7 | 2 | 5 | 6 | 6 | 5 | 2 | 7 | 7 | |
| 88 | 6 | 7 | 6 | 5 | 2 | 7 | 6 | 2 | 5 | 6 | 5 | 7 | 6 | 4 | 7 | 7 | 4 | 7 | 9 | 8 | 7 | 6 | 7 | 8 | 6 |
| | | | | | | | | | 1 | 2 | 5 | 7 | 2 | 2 | 7 | 7 | 2 | 6 | 8 | 7 | 7 | 2 | 7 | 8 | 5 |
| 89 | 6 | 4 | 6 | 7 | 2 | 7 | 6 | 4 | 5 | 6 | 5 | 8 | 7 | | 8 | 7 | 5 | 8 | 8 | 8 | 8 | 6 | 7 | 8 | 7 |
| | | | | | | | | | 1 | 2 | 2 | 7 | 2 | | 7 | 6 | 4 | 7 | 7 | 7 | 7 | | 7 | 7 | 6 |
| 90 | 3 | 2 | 7 | 6 | | 7 | 6 | | 5 | 4 | 6 | 7 | 4 | 2 | 8 | 8 | 4 | 7 | 8 | 8 | 8 | 4 | 7 | 7 | |
| | | | | | | | | | 1 | 2 | 2 | 5 | 2 | | 7 | 6 | 2 | 6 | 7 | 7 | 8 | | 7 | 6 | |
| 91 | 2 | | 4 | | | 7 | 2 | 2 | 5 | 7 | 7 | 7 | 7 | 7 | 8 | 8 | 7 | 7 | 9 | 7 | 6 | 8 | 7 | 7 | 8 |
| | | | | | | | | | 1 | 4 | 6 | 6 | 5 | 6 | 7 | 8 | 7 | 6 | 6 | 5 | 2 | 6 | 7 | 7 | 8 |
| 92 | 3 | 4 | 6 | 3 | 4 | 7 | 7 | 5 | 5 | 4 | 3 | 7 | 4 | 7 | 8 | 7 | 6 | 5 | 6 | 6 | 5 | 6 | 7 | 7 | 4 |
| | | | | | | | | | 1 | 2 | 2 | 6 | 2 | 6 | 7 | 7 | 5 | 1 | 2 | 2 | 2 | 2 | 6 | 6 | 2 |

TABLE IV (continued)

| Compound of | Soil drench 10 kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 93 | 7 | 6 | 5 | 4 | 7 | 6 | 5 | 6 | 5 | 2 | 4 | 7 | 3 | 7 | 8 | 8 | 6 | 7 | 6 | 5 | 6 | 7 | 7 | 7 | 4 |
| | | | | | | | | | 1 | | 2 | 5 | | 6 | 7 | 7 | 4 | 4 | | 2 | 2 | 6 | 6 | 6 | 2 |
| 94 | 5 | 6 | 4 | 1 | 1 | 7 | 7 | 5 | 5 | 4 | 7 | 8 | 7 | 7 | 8 | 8 | 6 | 7 | 8 | 8 | 4 | 5 | 7 | 7 | 4 |
| | | | | | | | | | 1 | 2 | 6 | 7 | 2 | 6 | 7 | 8 | 5 | 4 | 6 | 6 | 2 | 2 | 7 | 6 | 3 |
| 95 | 6 | 4 | 4 | 4 | 7 | 7 | 7 | 4 | 5 | 5 | 4 | 7 | 4 | 7 | 8 | 8 | 6 | 4 | 6 | 7 | 6 | 6 | 7 | 7 | 4 |
| | | | | | | | | | 1 | 1 | 2 | 6 | | 7 | 8 | 7 | 4 | 2 | 5 | 2 | 5 | 2 | 6 | 7 | |
| 96 | 3 | 2 | 4 | 2 | 3 | 6 | 6 | 4 | 5 | 7 | 5 | 7 | 7 | 7 | 7 | 8 | 6 | 7 | 6 | 4 | 4 | 5 | 7 | 7 | 5 |
| | | | | | | | | | 1 | 2 | 2 | 4 | 2 | 6 | 7 | 7 | 5 | 2 | 2 | 2 | | 2 | 7 | 7 | 4 |
| 97 | 7 | 7 | 6 | 5 | 6 | 7 | 7 | 5 | 5 | 7 | 6 | 7 | 7 | 7 | 8 | 9 | 5 | 9 | 9 | 8 | 8 | 7 | 7 | 7 | 5 |
| | | | | | | | | | 1 | 5 | 5 | 6 | 5 | 6 | 7 | 8 | 4 | 7 | 8 | 6 | 6 | 6 | 7 | 6 | 2 |
| 98 | 7 | 8 | 7 | 6 | 6 | 7 | 8 | 6 | 5 | 7 | 7 | 8 | 7 | 7 | 8 | 9 | 7 | 8 | 9 | 8 | 8 | 7 | 7 | 8 | 8 |
| | | | | | | | | | 1 | 6 | 7 | 8 | 5 | 6 | 7 | 8 | 6 | 7 | 8 | 7 | 5 | 6 | 7 | 7 | 6 |
| 99 | * | * | * | * | * | * | * | * | 5 | 6 | 4 | 7 | 5 | 2 | 8 | 9 | 6 | 7 | 8 | 8 | 6 | | 7 | 8 | 7 |
| | | | | | | | | | 1 | 4 | 2 | 6 | 2 | | 8 | 8 | 6 | 5 | 7 | 7 | 2 | | 7 | 7 | 2 |
| 100 | 7 | 6 | 7 | 7 | 6 | 6 | 7 | 5 | 5 | 7 | 6 | 8 | 6 | 7 | 8 | 8 | 5 | 8 | 9 | 8 | 7 | 7 | 7 | 7 | 8 |
| | | | | | | | | | 1 | 6 | 6 | 7 | 4 | 7 | 7 | 7 | 4 | 6 | 9 | 7 | 6 | 6 | 7 | 7 | 7 |
| 101 | 7 | 6 | 7 | 6 | | 7 | 6 | 5 | 5 | 7 | 5 | 8 | 4 | 1 | 8 | 8 | 7 | 8 | 8 | 8 | 8 | | 7 | 7 | 7 |
| | | | | | | | | | 1 | 4 | 4 | 7 | 2 | | 7 | 7 | 5 | 6 | 7 | 6 | 6 | | 7 | 7 | 2 |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 102 | 7 | 7 | 6 | 6 |  | 8 | 6 | 5 | 5 | 7 | 6 | 7 | 4 | 3 | 8 | 8 | 6 | 7 | 9 | 9 | 6 | 4 | 7 | 7 | 5 |
|  |  |  |  |  |  |  |  |  | 1 | 2 | 2 | 7 | 2 | 1 | 7 | 7 | 5 | 6 | 2 | 6 | 5 |  | 6 | 5 |  |
| 103 | 7 | 2 | 6 | 7 |  | 9 | 7 | 3 | 5 | 4 | 3 | 7 | 3 |  | 9 | 8 | 5 | 6 | 2 | 8 | 7 | 5 | 7 | 7 | 2 |
|  |  |  |  |  |  |  |  |  | 1 | 2 |  | 5 |  |  | 8 | 6 | 4 | 2 |  | 6 | 6 | 2 | 5 | 5 |  |
| 104 | 6 | 7 | 6 | 6 | 2 | 7 | 7 | 2 | 5 | 6 | 7 | 7 | 5 | 5 | 7 | 7 | 6 | 5 | 6 | 6 | 7 | 6 | 8 | 7 | 2 |
|  |  |  |  |  |  |  |  |  | 1 | 2 | 6 | 7 | 2 | 5 | 7 | 6 | 4 | 2 | 5 | 5 | 7 | 2 | 7 | 7 |  |
| 105 | 7 | 7 | 7 | 7 | 5 | 8 | 7 | 7 | 5 | 8 | 7 | 8 | 8 | 6 | 8 | 8 | 8 | 8 | 9 | 8 | 7 | 8 | 8 | 8 | 5 |
|  |  |  |  |  |  |  |  |  | 1 | 5 | 7 | 8 | 7 | 2 | 7 | 7 | 6 | 7 | 8 | 5 | 6 | 5 | 8 | 7 | 2 |
| 106 | 6 | 7 | 7 | 4 | 7 | 8 | 7 | 6 | 5 | 6 | 7 | 7 | 4 | 6 | 8 | 8 | 4 | 8 | 9 | 7 | 7 | 8 | 8 | 8 | 8 |
|  |  |  |  |  |  |  |  |  | 1 | 5 | 6 | 5 | 2 | 6 | 7 | 7 | 2 | 6 | 7 | 6 | 6 | 7 | 8 | 7 | 6 |
| 107 | 7 | 4 | 7 | 2 | 4 | 7 | 6 | 4 | 5 | 3 | 6 | 6 | 4 | 6 | 8 | 8 | 7 | 4 | 7 | 8 | 2 | 2 | 7 | 8 | 2 |
|  |  |  |  |  |  |  |  |  | 1 | 1 | 2 | 6 |  | 5 | 8 | 7 | 2 | 2 | 2 | 4 |  |  | 6 | 8 |  |
| 108 | 3 | 7 | 6 | 2 | 2 | 8 | 7 | 2 | 5 | 4 | 7 | 7 | 2 | 4 | 9 | 8 | 4 | 7 | 7 | 6 | 4 |  | 7 | 8 | 2 |
|  |  |  |  |  |  |  |  |  | 1 | 2 | 7 | 7 |  |  | 8 | 7 |  | 4 | 6 | 6 |  |  | 5 | 7 |  |
| 109 | 7 | 7 | 6 | 7 | 4 | 8 | 7 | 6 | 5 | 7 | 6 | 8 | 7 | 6 | 9 | 9 | 8 | 8 | 9 | 8 | 8 | 7 | 8 | 8 | 8 |
|  |  |  |  |  |  |  |  |  | 1 | 6 | 5 | 7 | 7 | 5 | 8 | 8 | 7 | 7 | 9 | 7 | 6 | 6 | 7 | 7 | 4 |
| 110 | 8 | 8 | 4 | 3 | 8 | 9 | 9 | 8 | 5 | 6 | 7 | 7 | 7 | 8 | 9 | 9 | 7 | 4 | * | 7 | 4 | 8 | 8 | 8 | 8 |
|  |  |  |  |  |  |  |  |  | 1 | 1 | 6 | 7 | 5 | 8 | 9 | 8 | 6 |  | 2 | 3 |  | 6 | 8 | 8 | 6 |

TABLE IV (continued)

| Compound of | Soil drench 10 kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 111 | 7 | 8 | 8 | 7 | 7 | 8 | 8 | 7 | 5 | 7 | 8 | 8 | 6 | 8 | 9 | 9 | 9 | 7 | 9 | 8 | 7 | 7 | 8 | 8 | 8 |
| | | | | | | | | | 1 | 5 | 8 | 8 | 5 | 7 | 8 | 8 | 8 | 2 | 8 | 6 | 6 | 2 | 8 | 7 | 2 |
| 112 | 7 | 8 | 6 | 4 | 6 | 7 | 4 | 5 | 5 | 7 | 4 | 7 | 7 | 7 | 8 | 8 | 4 | 7 | 8 | 6 | 4 | 3 | 5 | 6 | 4 |
| | | | | | | | | | 1 | 6 | 2 | 7 | 5 | 7 | 8 | 7 | 2 | 2 | 7 | 2 | 2 | | 2 | 5 | 2 |
| 113 | 7 | 6 | 4 | 3 | 2 | 7 | 5 | 4 | 5 | 7 | 6 | 8 | 5 | 7 | 8 | 8 | 6 | 8 | 8 | 6 | 4 | 2 | 7 | 8 | 2 |
| | | | | | | | | | 1 | 4 | 5 | 7 | 4 | 6 | 8 | 8 | 5 | 2 | 7 | 4 | 2 | | 6 | 7 | |
| 114 | | | | | | | | | 5 | | | | | 4 | 6 | 4 | 5 | | | | | | | | |
| | | | | | | | | | 1 | | | | | 2 | 2 | 2 | 4 | | | | | | | | |
| 115 | 6 | 8 | 6 | 7 | | 8 | 8 | 6 | 5 | 4 | 7 | 7 | 2 | 4 | 9 | 8 | 6 | 4 | 8 | 8 | 4 | | 8 | 8 | 4 |
| | | | | | | | | | 1 | 2 | 6 | 7 | | 2 | 8 | 8 | 5 | 2 | 7 | 8 | 2 | | 8 | 8 | 2 |
| 116 | 7 | 8 | 6 | 5 | | 8 | 6 | 4 | 5 | 7 | 6 | 7 | 4 | 4 | 8 | 8 | 4 | 6 | 8 | 7 | 6 | 4 | 7 | 8 | 5 |
| | | | | | | | | | 1 | 2 | 5 | 7 | 2 | 2 | 7 | 7 | 2 | 5 | 6 | 6 | 2 | 2 | 6 | 7 | 4 |
| 117 | 7 | 6 | 6 | 4 | | 6 | 6 | 5 | 5 | 3 | 4 | 7 | 4 | | 8 | 8 | 6 | 6 | 7 | 6 | 2 | | 6 | 8 | 6 |
| | | | | | | | | | 1 | 1 | 2 | 6 | 2 | | 7 | 7 | 4 | 5 | 2 | 5 | | | 5 | 7 | 5 |
| 118 | 7 | 6 | 7 | 6 | 5 | 7 | 6 | 7 | 5 | 7 | 7 | 8 | 6 | 7 | 7 | 8 | 6 | 7 | 8 | 6 | 4 | 6 | 8 | 8 | 5 |
| | | | | | | | | | 1 | 4 | 7 | 8 | 2 | 2 | 6 | 8 | 5 | 2 | 7 | 5 | 2 | 2 | 7 | 7 | 4 |
| 119 | 7 | 8 | 7 | 7 | 7 | 8 | 8 | 8 | 5 | 8 | 8 | 8 | 7 | 8 | 8 | 8 | 8 | 8 | 9 | 8 | 4 | 8 | 8 | 8 | 7 |
| | | | | | | | | | 1 | 7 | 8 | 8 | 6 | 7 | 8 | 7 | 7 | 7 | 7 | 2 | 2 | 2 | 8 | 7 | 4 |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 120 | | | | | 2 | 8 | 7 | | 5 | | | | | | | | | | | | | | | | |
| | | | | | | | | | 1 | | | | | | | | | | | | | | | | |
| 121 | | | | | | 8 | 4 | | 5 | | | | | | 7 | 7 | 2 | | | | | | 7 | | |
| | | | | | | | | | 1 | | | | | | 6 | 5 | | | | | | | 7 | | |
| 122 | 7 | 8 | 7 | 7 | 4 | 8 | 7 | 6 | 5 | 7 | 8 | 8 | 7 | 7 | 8 | 8 | 8 | 4 | 8 | 7 | 6 | 4 | 8 | 8 | 2 |
| | | | | | | | | | 1 | 5 | 8 | 8 | 6 | 5 | 8 | 8 | 7 | 2 | 8 | 4 | 5 | | 8 | 7 | |
| 123 | 7 | 8 | 8 | 7 | 7 | 8 | 8 | 7 | 5 | 6 | 7 | 8 | 7 | 7 | 8 | 9 | 7 | 8 | 9 | 7 | 6 | 8 | 8 | 8 | 6 |
| | | | | | | | | | 1 | 5 | 7 | 8 | 6 | 6 | 7 | 8 | 6 | 4 | 8 | 6 | 5 | 2 | 8 | 7 | 5 |
| 124 | 4 | 7 | 7 | 4 | | 7 | 6 | 7 | 5 | 2 | 6 | 6 | | | 8 | 8 | 2 | 3 | 7 | 5 | 2 | | 8 | 8 | 2 |
| | | | | | | | | | 1 | | 5 | 2 | | | 7 | 7 | | | 6 | 4 | | | 7 | 6 | |
| 125 | 7 | 6 | 6 | 2 | 7 | 7 | 9 | 7 | 5 | 4 | 7 | 8 | 4 | 8 | 8 | 9 | 7 | 6 | 4 | 7 | 2 | 7 | 8 | 8 | 8 |
| | | | | | | | | | 1 | | 4 | 7 | 2 | 7 | 8 | 8 | 6 | | 2 | 2 | | 2 | 7 | 7 | 7 |
| 126 | | | | | | | | | 5 | | | | | | 4 | 2 | | | | | | | | | |
| | | | | | | | | | 1 | | | | | | 2 | | | | | | | | | | |
| 127 | 6 | 5 | 7 | 4 | | 7 | 8 | 6 | 5 | 2 | 1 | 8 | 1 | 2 | 8 | 8 | 4 | 2 | 1 | 4 | | | 8 | 8 | 4 |
| | | | | | | | | | 1 | | | 2 | | | 8 | 8 | 2 | | | 2 | | | 7 | 6 | 2 |
| 128 | 5 | 7 | 7 | 6 | | 8 | 8 | 2 | 5 | 7 | 5 | 8 | 3 | | 8 | 8 | 2 | 6 | 6 | 8 | 4 | | 8 | 7 | 2 |
| | | | | | | | | | 1 | 2 | 2 | 2 | | | 8 | 8 | | 5 | 4 | 7 | 2 | | 8 | 6 | |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 129 | 7 | 5 | 7 | 6 | 5 | 7 | 6 | 5 | 5 | 7 | 7 | 8 | 5 | 8 | 8 | 9 | 8 | 7 | 7 | 7 | 4 | 6 | 7 | 8 | 4 |
| | | | | | | | | | 1 | 2 | 6 | 7 | 4 | 8 | 8 | 8 | 6 | 2 | 2 | 2 | 2 | | 6 | 7 | 2 |
| 130 | 8 | 8 | 7 | 6 | 8 | 8 | 8 | 8 | 5 | 8 | 8 | 8 | 8 | 8 | 9 | 9 | 8 | 8 | 9 | 7 | 6 | 8 | 8 | 8 | 6 |
| | | | | | | | | | 1 | 5 | 8 | 8 | 5 | 8 | 8 | 9 | 8 | 4 | 7 | 4 | 5 | 4 | 8 | 6 | 5 |
| 131 | | | 4 | 2 | | 8 | 8 | 5 | 5 | | | 5 | 2 | 7 | 8 | 8 | 7 | 2 | 4 | 3 | 2 | | 7 | 7 | 5 |
| | | | | | | | | | 1 | | | 2 | | 2 | 8 | 7 | 6 | | 2 | | | | 7 | 5 | 4 |
| 132 | 6 | 7 | 6 | 7 | 7 | 7 | 8 | 5 | 5 | 5 | 7 | 8 | 2 | 6 | 8 | 8 | 6 | 4 | 8 | 7 | 4 | 7 | 8 | 8 | 5 |
| | | | | | | | | | 1 | * | * | * | * | * | * | * | * | * | * | * | * | * | * | * | * |
| 139 | 8 | 6 | 8 | 6 | 6 | 7 | 5 | 8 | 5 | 8 | 6 | 8 | 7 | 7 | 8 | 8 | 8 | 7 | 9 | 8 | 7 | 8 | 7 | 8 | 8 |
| | | | | | | | | | 1 | 7 | 4 | 7 | 6 | 6 | 8 | 6 | 7 | 5 | 8 | 7 | 5 | 5 | 7 | 8 | 7 |
| 140 | 3 | 7 | 6 | 7 | 7 | 8 | 4 | 7 | 5 | 7 | 7 | 8 | 6 | 8 | 8 | 8 | 7 | 5 | 9 | 5 | 6 | 8 | 8 | 8 | 8 |
| | | | | | | | | | 1 | 5 | 4 | 8 | 5 | 8 | 8 | 8 | 6 | 3 | 4 | 3 | 2 | 7 | 8 | 8 | 8 |
| 141 | 7 | 8 | 7 | 2 | 8 | 8 | 8 | 8 | 5 | 6 | 7 | 7 | 4 | 8 | 9 | 9 | 9 | 6 | 7 | 8 | | 8 | 8 | 8 | 9 |
| | | | | | | | | | 1 | 5 | 5 | 7 | 2 | 8 | 8 | 8 | 8 | 5 | 6 | 6 | | 6 | 8 | 8 | 9 |
| 142 | 8 | 8 | 7 | 6 | 7 | 8 | 8 | 8 | 5 | 8 | 8 | 8 | 6 | 8 | 8 | 8 | 8 | 5 | 8 | 8 | 7 | 8 | 8 | 8 | 8 |
| | | | | | | | | | 1 | 6 | 8 | 8 | 5 | 7 | 8 | 8 | 8 | | 7 | 7 | 6 | 8 | 8 | 8 | 7 |
| 143 | 8 | 8 | 6 | 4 | 7 | 8 | 7 | 7 | 5 | 8 | 8 | 8 | 2 | 8 | 9 | 8 | 8 | 7 | 8 | 3 | 2 | 7 | 8 | 8 | 7 |
| | | | | | | | | | 1 | 8 | 7 | 8 | 2 | 8 | 8 | 7 | 8 | 2 | 4 | 2 | 1 | 5 | 8 | 7 | 5 |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 144 | 7 | 7 | 6 | 5 | 2 | 8 | 4 | 8 | 5 | 7 | 8 | 8 | 6 | 7 | 8 | 8 | 7 | 7 | 8 | 7 | 4 | 5 | 8 | 8 | 8 |
| | | | | | | | | | 1 | 4 | 8 | 8 | 5 | 7 | 8 | 8 | 7 | 6 | 7 | 6 | 2 | 4 | 8 | 8 | 8 |
| 145 | 6 | 5 | 4 | 6 | 5 | 7 | 4 | 6 | 5 | 6 | 7 | 8 | 5 | 7 | 8 | 8 | 5 | 4 | 4 | 4 | 2 | 3 | 8 | 8 | 6 |
| | | | | | | | | | 1 | 5 | 7 | 7 | 4 | 7 | 8 | 8 | 4 | 4 | 2 | 2 | | | 7 | 8 | 5 |
| 146 | 7 | 7 | 8 | 6 | 8 | 8 | 9 | 7 | 5 | 7 | 7 | 8 | 4 | 8 | 9 | 8 | 7 | 8 | 8 | 8 | 4 | 8 | 8 | 8 | 7 |
| | | | | | | | | | 1 | 6 | 5 | 8 | 1 | 7 | 8 | 7 | 7 | 6 | 7 | 6 | 2 | 6 | 8 | 8 | 7 |
| 147 | 7 | 7 | 8 | 6 | 8 | 8 | 8 | 7 | 5 | 7 | 7 | 8 | 2 | 8 | 8 | 8 | 8 | 8 | 9 | 8 | 7 | 8 | 8 | 8 | 8 |
| | | | | | | | | | 1 | 4 | 6 | 7 | 2 | 7 | 7 | 8 | 7 | 4 | 8 | 6 | 5 | 6 | 8 | 7 | 7 |
| 148 | 8 | 8 | 7 | 7 | 8 | 8 | 8 | 7 | 5 | 8 | 7 | 8 | 6 | 8 | 9 | 9 | 8 | 8 | 9 | 8 | 7 | 8 | 8 | 8 | 8 |
| | | | | | | | | | 1 | 6 | 6 | 8 | 2 | 8 | 8 | 8 | 7 | 6 | 8 | 8 | 5 | 7 | 8 | 8 | 7 |
| 149 | 8 | 7 | 8 | 7 | 8 | 8 | 8 | 7 | 5 | 7 | 7 | 9 | 8 | 8 | 9 | 9 | 8 | 7 | 9 | 8 | 6 | 6 | 7 | 7 | 5 |
| | | | | | | | | | 1 | 7 | 7 | 8 | 7 | 8 | 8 | 8 | 7 | 7 | 8 | 7 | 6 | 6 | 7 | 7 | 4 |
| 150 | 6 | 7 | 7 | 6 | 8 | 8 | 8 | 7 | 5 | 6 | 5 | 9 | 7 | 8 | 8 | 8 | 8 | 8 | 8 | 7 | 7 | 6 | 7 | 7 | 7 |
| | | | | | | | | | 1 | 4 | 2 | 7 | 6 | 8 | 8 | 8 | 7 | 7 | 7 | 6 | 7 | 5 | 7 | 7 | 7 |
| 151 | 7 | 6 | 7 | 6 | 7 | 8 | 8 | 7 | 5 | 4 | 6 | 8 | 7 | 8 | 8 | 8 | 7 | 8 | 7 | 6 | 2 | 8 | 8 | 8 | 8 |
| | | | | | | | | | 1 | 2 | 2 | 7 | 5 | 7 | 8 | 8 | 7 | 5 | 2 | 5 | | 7 | 8 | 8 | 7 |
| 152 | 7 | 6 | 7 | 5 | 6 | 8 | 7 | 6 | 5 | 7 | 7 | 9 | 7 | 7 | 8 | 8 | 8 | 7 | 8 | 7 | 6 | 5 | 6 | 7 | 4 |
| | | | | | | | | | 1 | 6 | 6 | 8 | 6 | 7 | 7 | 8 | 6 | 5 | 7 | 6 | 6 | 4 | 5 | 7 | 2 |

TABLE IV (continued)

| Compound of Ex. No. | Soil drench 10 kg/ha Mz | R | BG | O | L | M | SB | S | Dosage kg/ha | Foliar spray Mz | R | BG | O | L | M | SB | S | Pre-emergence Mz | R | BG | O | L | M | SB | S |
|---|---|---|---|---|---|---|---|---|---|---|---|---|---|---|---|---|---|---|---|---|---|---|---|---|---|
| 153 | 7 | 7 | 6 | 7 | 7 | 8 | 8 | 6 | 5 | 7 | 7 | 8 | 6 | 8 | 8 | 9 | 8 | 7 | 5 | 7 | 5 | 4 | 6 | 5 | 2 |
| | | | | | | | | | 1 | 5 | 7 | 7 | 5 | 8 | 7 | 7 | 7 | 6 | 4 | 6 | 4 | | 5 | 2 | |
| 159 | 7 | 7 | 6 | 5 | 4 | 7 | 4 | 6 | 5 | 8 | 8 | 9 | 8 | 8 | 8 | 8 | 8 | 8 | 9 | 7 | 4 | 7 | 8 | 8 | 8 |
| | | | | | | | | | 1 | 6 | 8 | 8 | 7 | 7 | 8 | 8 | 7 | 2 | 7 | 5 | 2 | 2 | 8 | 7 | 7 |
| 160 | 8 | 8 | 8 | 7 | 8 | 8 | 9 | 7 | 5 | 7 | 7 | 8 | 7 | 8 | 8 | 8 | 8 | 9 | 9 | 9 | 8 | 8 | 8 | 8 | 8 |
| | | | | | | | | | 1 | 7 | 7 | 8 | 7 | 8 | 7 | 8 | 7 | 8 | 8 | 8 | 4 | 7 | 8 | 8 | 8 |
| 161 | 7 | 7 | 8 | 6 | 8 | 8 | 9 | 7 | 5 | 7 | 7 | 8 | 6 | 8 | 8 | 9 | 8 | 8 | 8 | 9 | 4 | 7 | 8 | 8 | 9 |
| | | | | | | | | | 1 | 6 | 6 | 7 | 5 | 8 | 8 | 8 | 7 | 7 | 6 | 8 | 2 | 6 | 8 | 8 | 7 |

## Claims

1. A compound of the general formula

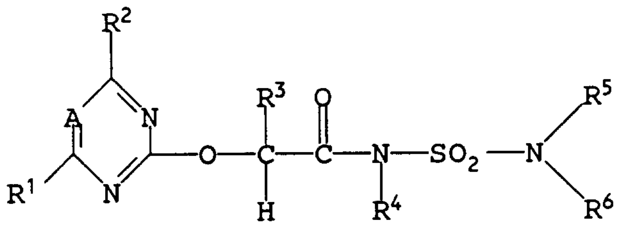

(I)

in which

A represents a nitrogen atom or a group $CR^7$;

$R^1$, $R^2$ and $R^7$ each independently represents a hydrogen or halogen atom, a formyl, cyano, carboxy or azido group, or an optionally substituted alkyl, alkenyl, alkynyl, cycloalkyl, alkoxy, alkenyloxy, alkynyloxy, aryloxy, alkylthio, alkenylthio, alkynylthio, arylthio, alkylcarbonyl, alkoxycarbonyl, amino. aminoxy or dialkyliminoxy group;

$R^3$ represents a hydrogen atom, or an optionally substituted alkyl, alkenyl, alkynyl, cycloalkyl, heterocyclic, aralkyl or aryl group;

$R^4$ represents a hydrogen atom or an optionally substituted alkyl, alkenyl, aralkyl, aryl or heterocyclic group, or an optionally substituted acyl group of the formula $COR^8$ in which $R^8$ represents an alkyl, aryl or aralkyl group; and

$R^5$ and $R^6$ each independently represents a hydrogen atom or an optionally substituted alkyl, alkoxy, alkenyl, alkynyl, aryl, aralkyl, amino, cycloalkyl or heterocyclic group, or a group of the formula $SO_2R^8$, in which $R^8$ is as defined hereinabove, or a group

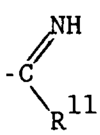

,

in which $R^{11}$ is an alkylthio group, or together form a group

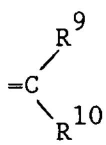

in which $R^9$ and $R^{10}$ each independently represents a hydrogen atom or an alkyl, alkoxy, aryl, aralkyl or dialkylamino group, or $R^9$ and $R^{10}$ together form an optionally substituted heterocyclic group, or $R^5$ and $R^6$ together form an alkylene chain which is optionally interrupted by an oxygen or sulphur atom or by a group -NR- in which R represents a hydrogen atom or an alkyl group;

or a salt thereof.

2. A compound as claimed in claim 1, in which A represents a nitrogen atom or a group CH.

3. A compound as claimed in claim 1 or claim 2, in which $R^1$ represents a $C_{1-4}$ alkyl, $C_{1-4}$ alkoxy or $C_{1-4}$ haloalkyl group.

4. A compound as claimed in claim 1, 2 or 3, in which $R^2$ represents a $C_{1-4}$ alkyl or $C_{1-4}$ alkoxy group or a halogen atom.

5. A compound as claimed in any one of claims 1 to 4, in which $R^3$ represents a $C_{1-6}$ alkyl or a phenyl group.

6. A compound as claimed in any one of claims 1 to 5, in which $R^4$ represents a hydrogen atom.

**7.** A compound as claimed in any one of claims 1 to 6, in which $R^5$ and $R^6$ each independently represents a hydrogen atom, a $C_{1-6}$ alkyl, $C_{1-6}$ haloalkyl, $C_{1-6}$ alkoxy, $C_{2-6}$ alkenyl, $C_{2-6}$ alkynyl, mono- or di-($C_{1-4}$ alkoxy)$C_{1-4}$ alkyl, ($C_{1-4}$ alkoxy)carbonyl($C_{1-4}$ alkyl), $C_{3-8}$ cycloalkyl or benzyl group, or an optionally substituted phenyl, pyridyl, pyrimidinyl or ($C_{3-8}$ cycloalkyl) $C_{1-4}$ alkyl group, or a group

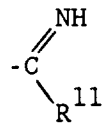

in which $R^{11}$ is a $C_{1-4}$ alkylthio group, or together form a group

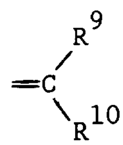

in which $R^9$ and $R^{10}$ each independently represents a $C_{1-4}$ alkyl, $C_{1-4}$ alkoxy or di($C_{1-4}$ alkyl)amino group, or $R^9$ and $R^{10}$ together form a five-membered ring in which two or three ring members are hetero atoms selected from nitrogen and sulphur atoms, the ring being substituted by a benzyl or one or two $C_{1-4}$ alkyl groups, or $R^5$ and $R^6$ together form an alkylene chain which is optionally interrupted by an oxygen atom or by a group -NR- in which R represents a $C_{1-4}$ alkyl group.

**8.** A compound as claimed in any one of claims 1 to 7, in which $R^1$ represents a methyl, methoxy or trifluoromethyl group; $R^2$ represents a methyl or methoxy group or a chlorine atom; $R^3$ represents a methyl, ethyl, n-propyl, i-propyl, n-butyl, i-butyl, s-butyl, t-butyl or phenyl group; $R^4$ represents a hydrogen atom; $R^5$ represents a hydrogen atom or a methyl or ethyl group; $R^6$ represents a hydrogen atom or a methyl, ethyl, n-propyl, i-propyl, n-butyl, methoxy, chloroethyl, methoxycarbonylmethyl mono- or dimethoxyethyl, allyl, propynyl, cyclopropyl, cyclobutyl, pyridyl, dimethylpyrimidinyl, (dichlorocyclopropyl)methyl, phenyl,
chlorophenyl or benzyl group or a group

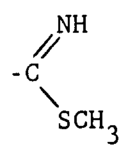

,

or $R^5$ and $R^6$ together represent one of the groups

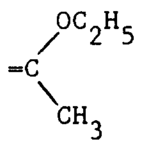 , 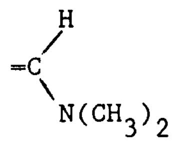 , 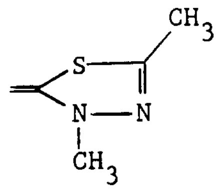 ,

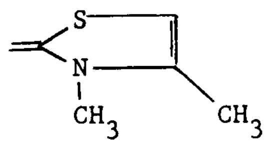 ,

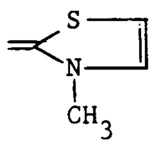 and 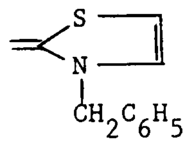 ,

or $R^5$ and $R^6$ together represent a group $-(CH_2)_2O(CH_2)_2-$, $-(CH_2)_2N(CH_3)(CH_2)_2-$ or $-(CH_2)_4-$.

**9.** A process for the preparation of a compound as claimed in claim 1, which comprises

(a) reacting a compound of the general formula

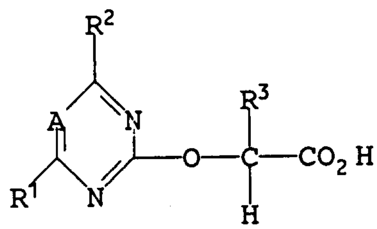 (II)

in which A, $R^1$, $R^2$ and $R^3$ are as defined in claim 1, or a corresponding ester, acid chloride or acid anhydride, with a compound of the general formula

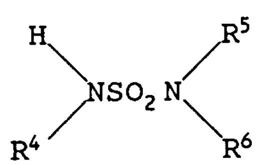 (III)

in which $R^4$, $R^5$ and $R^6$ are as defined in claim 1, or a salt thereof, if appropriate in the presence of a carboxyl-activating agent, or

(b) reacting a compound of the general formula

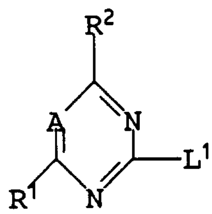

(IV)

in which A, $R^1$ and $R^2$ are as defined in claim 1, and $L^1$ represents a leaving group, with, when $R^4$ represents a hydrogen atom, a di-salt, and, when $R^4$ represents a moiety other than a hydrogen atom, a mono-salt of a compound of the general formula

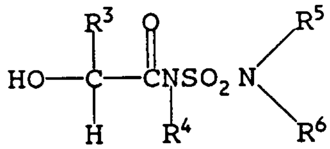

(V)

in which $R^3$, $R^4$, $R^5$ and $R^6$ are as defined in claim 1,

and, if required or desired, converting a resulting compound into another compound as claimed in claim 1.

**10.** A herbicidal composition comprising a compound as claimed in any one of claims 1 to 8, together with a carrier and/or surface-active agent.

**11.** A method of combating undesired plant growth at a locus, which comprises treating the locus with a compound of formula I as claimed in any one of claims 1 to 8, or with a composition as claimed in claim 10.

European Patent Office

# EUROPEAN SEARCH REPORT

Application Number

EP 92 20 4080

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 411 706 (SHELL INTERNATIONALE RESEARCH MAATSCHAPPIJ B.V.) 6 February 1991<br>*see whole document*<br>--- | 1-11 | C07D239/60<br>C07D239/553<br>C07D239/34<br>C07D251/30<br>C07D251/20<br>C07D417/12<br>C07D401/12<br>A01N43/54<br>A01N43/66 |
| A | EP-A-0 431 707 (SHELL INTERNATIONALE RESEARCH MAATSCHAPPIJ B.V.) 12 June 1991<br>--- | 1-11 | |
| A | EP-A-0 336 494 (SHELL INTERNATIONALE RESEARCH MAATSCHAPPIJ B.V.) 11 October 1989<br>--- | 1-11 | |
| Y | CHEMICAL ABSTRACTS, vol. 116, no. 7, 17 February 1992, Columbus, Ohio, US; abstract no. 59390U, 'preparation of pyrimidinyloxy-, pyrimidinylthio- or pyrimidinyliminoalkanamides as herbicides'<br>page 865 ;<br>*and JP03200772, published 02-09-91*<br>--- | 1-11 | |
| A | CHEMICAL ABSTRACTS, vol. 115, no. 25, 23 December 1991, Columbus, Ohio, US; abstract no. 280053N, 'preparation of 2-(hetero)arylthio-2-(azinylthio or oxy-)acetates as herbicides'<br>page 1020 ;<br>* and JP3193765 published 23-08-91*<br>----- | 1-11 | TECHNICAL FIELDS SEARCHED (Int. Cl.5)<br>C07D<br>A01N |

The present search report has been drawn up for all claims

| Place of search | Date of completion of the search | Examiner |
|---|---|---|
| MUNICH | 11 FEBRUARY 1993 | SCRUTON-EVANS I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)